# EUROPEAN PATENT APPLICATION

(11) **EP 4 050 615 A1**
(43) Date of publication of application: **31.08.2022**
(21) Application number: 22151979.6
(22) Date of filing: 18.01.2022
(51) Int. Cl.: G16H 10/40, G16H 40/40, G16H 40/63, G16H 40/67

(54) **CONTROL METHOD FOR SPECIMEN ANALYSIS SYSTEM, SPECIMEN ANALYSIS SYSTEM, COMPUTER, AND PROGRAM**

(30) Priority: 26.02.2021 JP 2021030891
(71) Applicant: SYSMEX CORPORATION, Kobe-shi Hyogo 651-0073 (JP)
(72) Inventor: Yoneda, Takurou, Kobe-shi, Hyogo, 651-0073 (JP); Nishikawa, Ken, Kobe-shi, Hyogo, 651-0073 (JP)
(74) Representative: Hoffmann Eitle

(57) **Abstract**

Disclosed is a control method for a specimen analysis system including an analyzer configured to analyze a specimen, the control method including: controlling, by a first computer, the analyzer; transmitting, by the first computer, an analysis result to a second computer communicably connected to the first computer via a network and configured to manage the analysis result; transmitting, by the first computer, setting information about a control program for the analyzer and setting information about communication with the second computer, to a third computer communicably connected to the first computer; storing, by the third computer, the setting information about the control program and the setting information about the communication which have been transmitted from the first computer; and controlling, by the third computer instead of the first computer, the analyzer on the basis of the stored setting information about the control program, and transmitting, by the third computer instead of the first computer, an analysis result to the second computer on the basis of the stored setting information about the communication.

## Description

### CROSS REFERENCE TO RELATED APPLICATIONS

This application claims priority from prior Japanese Patent Application No. 2021-030891, filed on February 26, 2021, entitled "CONTROL METHOD FOR SPECIMEN ANALYSIS SYSTEM, SPECIMEN ANALYSIS SYSTEM, COMPUTER, AND PROGRAM", the entire content of which is incorporated herein by reference.

### FIELD OF THE INVENTION

The present invention relates to a control method for a specimen analysis system, the specimen analysis system, a computer, and a program.

### BACKGROUND

Japanese Laid-Open Patent Publication No. 2002-90369 discloses a configuration in which, in a specimen analysis system having two automatic analyzers and two operation portions connected in the same local area network, unique-to-device information stored in a main operation portion such as calibration curve information and reagent remaining amount information is backed up on a storage medium of a sub operation portion. The main operation portion and the sub operation portion are used as man-machine interfaces with the automatic analyzers in order to, for example, input an analysis item for each specimen and input an instruction to start or stop analysis. If an abnormality occurs in the main operation portion, setting of the sub operation portion connected over the same network as that of the main operation portion is changed such that the sub operation portion can be used as the main operation portion. Thus, the sub operation portion performs, instead of the main operation portion, a process that was performed by the main operation portion.

If an abnormality occurs in the main operation portion, specimen analysis cannot be continued. Thus, the above change of the setting of the sub operation portion needs to be swiftly performed. However, the need for the switching to the sub operation portion upon occurrence of an abnormality in the main operation portion does not frequently arise, and thus a user of the specimen analysis system is ordinarily inexperienced in the above work of changing the setting of the sub operation portion and requires a long time for the work. In the above Japanese Laid-Open Patent Publication No. 2002-90369, the setting of the sub operation portion needs to be changed in the case of occurrence of an abnormality in the main operation portion such that the sub operation portion can be used as the main operation portion, but no specific method for the change is disclosed.

### SUMMARY OF THE INVENTION

The present invention is directed to shortening a time required for work of switching from a computer to an alternative computer in a specimen analysis system.

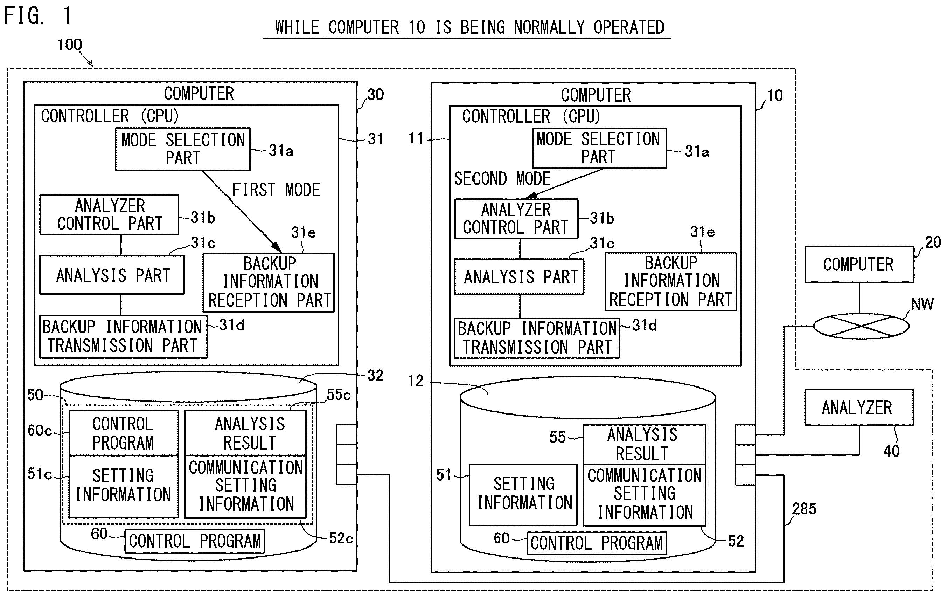

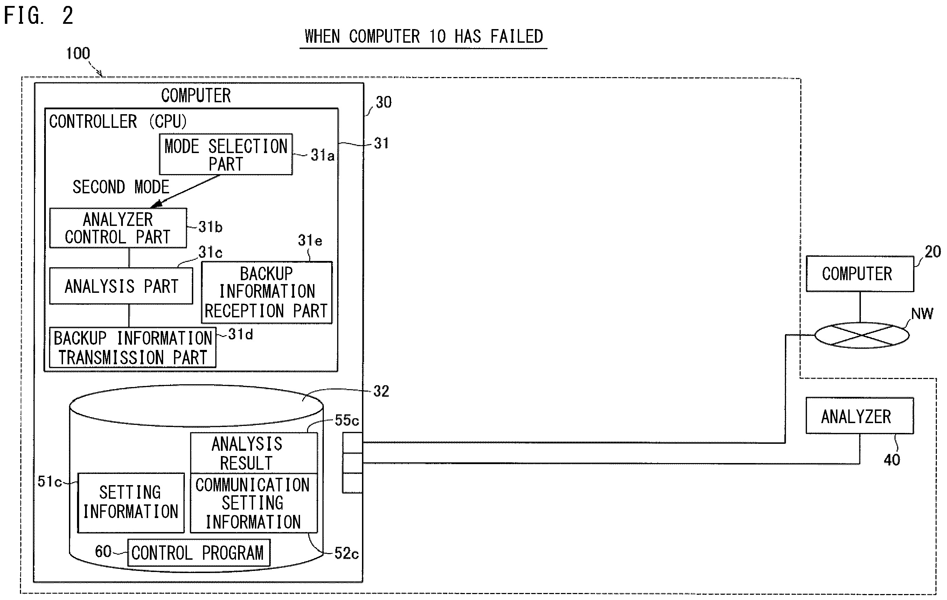

In order to achieve the above object, a control method for a specimen analysis system (100) of the present invention is, as shown in FIG. 1 and FIG. 2, a control method for a specimen analysis system (100) including an analyzer (40) configured to analyze a specimen. The control method includes: controlling, by a first computer (10), the analyzer (40); transmitting, by the first computer (10), an analysis result to a second computer (20) communicably connected to the first computer (10) via a network and configured to manage the analysis result; transmitting, by the first computer (10), setting information (51c) about a control program (60) for the analyzer (40) and setting information (52c) about communication with the second computer (20), to a third computer (30) communicably connected to the first computer (10); storing, by the third computer (30), the setting information (51c) about the control program (60) and the setting information (52c) about the communication which have been transmitted from the first computer (10); and controlling, by the third computer (30) instead of the first computer (10), the analyzer (40) on the basis of the stored setting information (51c) about the control program (60), and transmitting, by the third computer (30) instead of the first computer (10), an analysis result to the second computer (20) on the basis of the stored setting information (52c) about the communication.

In the control method for the specimen analysis system (100) of the present invention, when the first computer (10) connected to the analyzer (40) has failed, the third computer (30) controls the analyzer (40) on the basis of the prestored setting information (51c) about the control program (60) and communicates with the second computer (20) on the basis of the prestored setting information (52c) about the communication. Consequently, the time required for work of switching from the computer (10) to the alternative computer (30) can be shortened in the specimen analysis system (100).

A specimen analysis system (100) of the present invention, as shown in FIG. 1 and FIG. 2, includes: an analyzer (40) configured to analyze a specimen; a first computer (10) configured to control the analyzer (40) and transmit, via a network, an analysis result to a second computer (20) configured to manage the analysis result; and a third computer (30) communicably connected to the first computer (10). The first computer (10) transmits, to the third computer (30), setting information (51c) about a control program (60) for the analyzer (40) and setting information (52c) about communication with the second computer (20). The third computer (30) stores therein the setting information (51c) about the control program (60) and the setting information (52c) about the communication which have been transmitted from the first computer (10). The third computer (30) instead of the first computer (10) controls the analyzer (40) on the basis of the stored setting information (51c) about the control program (60) and transmits an analysis result to the second computer (20) on the basis of the stored setting information (52c) about the communication.

In the specimen analysis system (100) of the present invention, when the first computer (10) has failed, the third computer (30) controls the analyzer (40) on the basis of the prestored setting information about the control program and communicates with the second computer (20) on the basis of the prestored setting information about the communication. Consequently, the time required for work of switching from the computer (10) to the alternative computer (30) can be shortened in the specimen analysis system (100).

A control method for a specimen analysis system of the present invention is, as shown in FIG. 1 and FIG. 2, a backup method for executing, by a computer (30), controlling of an analyzer (40) and obtaining of backup information (50) about the analyzer (40). The backup method includes: selecting a first mode of obtaining the backup information (50) corresponding to information stored in another computer (10) connected to the analyzer (40), the other computer (10) being configured to control the analyzer (40), or a second mode of controlling the analyzer (40); obtaining the backup information (50) from the other computer (10) if the first mode is selected; and controlling the analyzer (40) if the second mode is selected.

In the control method for the specimen analysis system of the present invention, the computer (30) is operated in the first mode to obtain the backup information (50) while the other computer (10) configured to control the analyzer (40) is normally functioning. If the mode of the computer (30) is changed to the second mode, the computer (30) instead of the other computer (10) configured to control the analyzer (40) can control the analyzer (40) by using the prestored backup information (50). Consequently, the time required for work of switching from the other computer (10) to the alternative computer (30) can be shortened in the specimen analysis system (100).

A computer (30) of the present invention is, as shown in FIG. 1 and FIG. 2, a computer (30) configured to execute controlling of an analyzer (40) and obtaining of backup information (50) about the analyzer (40). The computer (30) includes a controller (31). The controller (31) executes: selecting a first mode of obtaining the backup information (50) corresponding to information stored in another computer (10) connected to the analyzer (40), the other computer (10) being configured to control the analyzer (40), or a second mode of controlling the analyzer (40); obtaining the backup information (50) from the other computer (10) if the first mode is selected; and controlling the analyzer (40) if the second mode is selected.

By changing, to the second mode, the mode of the computer (30) which is operated in the first mode and has prestored therein the backup information (50), the computer (30) of the present invention can control the analyzer (40) by using the prestored backup information (50) instead of the other computer (10) configured to control the analyzer (40). Consequently, the time required for work of switching from the other computer (10) to the alternative computer (30) can be shortened in the specimen analysis system (100).

A program (60) of the present invention is, as shown in FIG. 1 and FIG. 2, a program (60) configured to cause controlling of an analyzer (40) and obtaining of backup information (50) about the analyzer (40). The program (60) is configured to cause a computer (30) to execute: selecting a first mode of obtaining the backup information (50) corresponding to information stored in another computer (10) connected to the analyzer (40), the other computer (10) being configured to control the analyzer (40), or a second mode of controlling the analyzer (40); obtaining the backup information (50) from the other computer (10) if the first mode is selected; and controlling the analyzer (40) if the second mode is selected.

By changing, to the second mode, the mode of the computer (30) which is operated in the first mode and has prestored therein the backup information (50), the program (60) of the present invention enables the computer (30) to control the analyzer (40) by using the prestored backup information (50) instead of the other computer (10) configured to control the analyzer (40). Consequently, the time required for work of switching from the other computer (10) to the alternative computer (30) can be shortened in the specimen analysis system (100).

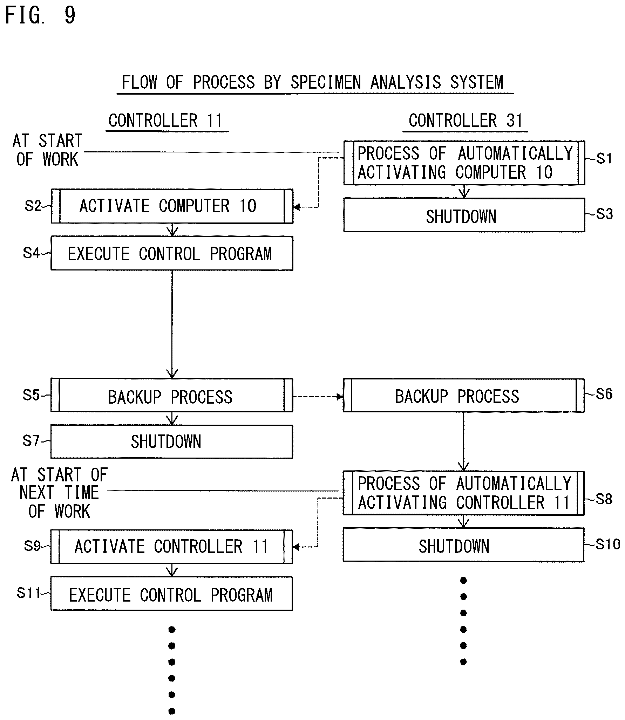

A control method for a specimen analysis system of the present invention is, as shown in FIG. 1 and FIG. 9, a control method for a specimen analysis system (100), the control method being for executing, by a backup computer (30), obtaining of backup information (50) about controlling of an analyzer (40). The control method includes: activating a main computer (10) connected to the analyzer (40) and configured to control the analyzer (40); and obtaining, from the activated main computer (10), the backup information (50) corresponding to information stored in the main computer (10).

Consequently, the backup computer (30) having stored therein the backup information (50) enables activation of the main computer (10) configured to control the analyzer (40). Thus, the main computer (10) can be automatically activated according to the convenience of a user without separately providing a computer for activating the main computer (10).

A specimen analysis system (100) of the present invention is, as shown in FIG. 1 and FIG. 9, a specimen analysis system configured to execute, by a backup computer (30), obtaining of backup information (50) about controlling of an analyzer (40). The specimen analysis system (100) includes: an analyzer (40) configured to analyze a specimen; a main computer (10) configured to control the analyzer (40); and a backup computer (30) communicably connected to the main computer (10). The backup computer (30) includes a controller (31). The controller (31) executes: activating the main computer (10); and obtaining, from the activated main computer (10), the backup information (50) corresponding to information stored in the main computer (10).

Consequently, the backup computer (30) having stored therein the backup information (50) enables activation of the main computer (10) configured to control the analyzer (40). Thus, the main computer (10) can be automatically activated according to the convenience of a user without separately providing a computer for activating the main computer (10).

According to the present invention, the time required for work of switching from a computer to an alternative computer can be shortened in the specimen analysis system.

### BRIEF DESCRIPTION OF THE DRAWINGS

FIG. 1 is a block diagram showing a specimen analysis system in which a first computer is being normally operated;
FIG. 2 is a block diagram showing the specimen analysis system in which the first computer has failed;
FIG. 3 is a schematic front view of the specimen analysis system;
FIG. 4 is a schematic plan view of the specimen analysis system;
FIG. 5 is a block diagram showing the mutual connection relationship between units which compose the specimen analysis system;
FIG. 6 is a schematic diagram showing a configuration example of measurement units and a transport unit;
FIG. 7 is a block diagram for explaining a hardware configuration of a computer;
FIG. 8 is a diagram for explaining backup information;
FIG. 9 is a flowchart for explaining an operation of the specimen analysis system;
FIG. 10A is a flowchart showing processing to be performed by a controller of a computer during a backup process;
FIG. 10B is a flowchart showing processing to be performed by a controller of a backup computer during the backup process;
FIG. 11A is a flowchart showing processing to be performed through a communication interface during an automatic activation process for the computer;
FIG. 11B is a flowchart showing processing to be performed by the controller of the backup computer during the automatic activation process;
FIG. 12 is a diagram showing a GUI including a setting screen for a first schedule;
FIG. 13 is a diagram showing a GUI including a setting screen for a second schedule;
FIG. 14 is a diagram showing a setting screen in which setting of backup-target information is performed;
FIG. 15 is a system configuration diagram showing a state where exchange between a computer and a backup computer is made when the computer has failed;
FIG. 16 is a flowchart for explaining a process of switching the mode of the backup computer from a first mode to a second mode; and
FIG. 17 is a diagram showing a screen in which selection of the second mode is received.

### DETAILED DESCRIPTION OF THE PREFERRED EMBODIMENTS

Hereinafter, an embodiment will be described with reference to the drawings.

### [Overviews of Specimen Analysis System and Control Method for Specimen Analysis System]

Firstly, overviews of a specimen analysis system 100 and a control method for the specimen analysis system 100 according to the present embodiment will be described with reference to FIG. 1 and FIG. 2.

### (Specimen Analysis System)

As shown in FIG. 1, the specimen analysis system 100 includes an analyzer 40, a first computer 10, and a third computer 30. The first computer 10 can communicate with a second computer 20 via a network NW A specimen is a sample derived from an organism. The specimen is collected from a subject such as a patient. The specimen analysis system 100 performs analysis on the specimen and generates an analysis result of the specimen.

The analyzer 40 analyzes the specimen. One analyzer 40 or a plurality of analyzers 40 are provided in the specimen analysis system 100. Under the control of the computer 10, each of the analyzers 40 executes a measurement operation on a detection-target component in the specimen. The analyzer 40 outputs a measurement result of the detection-target component to the computer 10.

The computer 10 is a main computer that controls the one analyzer 40 or the plurality of analyzers 40. One computer 10 or a plurality of computers 10 are provided in the specimen analysis system 100. Each of the computers 10 obtains the measurement result, of the detection-target component in the specimen, that has been outputted from the analyzer 40. The computer 10 analyzes the acquired measurement result of the detection-target component and generates an analysis result of the specimen. The computer 10 transmits, via the network NW, the analysis result to the computer 20 which manages the analysis result.

The computer 20 receives and stores therein the analysis result transmitted from the computer 10. The computer 20 is, for example, a host computer that manages specimen information in the specimen analysis system 100. Each of individual specimens to be analyzed in the specimen analysis system 100 is provided with a specimen ID that is unique identification information. In the specimen analysis system 100, the individual specimen, information about the subject from whom the specimen has been derived, and information about an analysis result are managed so as to be associated with one another on the basis of the specimen ID. The computer 20 manages the specimen ID, a measurement order for the specimen, the information about the subject, the information about the analysis result, and the like.

The computer 30 is communicably connected to the computer 10. The computer 30 is a backup computer that functions as a main computer instead of the computer 10 when the computer 10 which is a main computer has failed. In the following description, the computer 30 which is functioning as the backup computer is sometimes referred to as a backup computer 30. Examples of the case where the computer 30 instead of the computer 10 performs controlling of the analyzer 40 and transmission of an analysis result to the computer 20, include: a case where the computer 10 has failed; and furthermore, a case where the operation of the computer 10 has slowed down, and obstruction of an examination task being performed by the specimen analysis system 100 is happening or there is a concern that the obstruction might happen.

### (Configurations of Main Computer and Backup Computer)

The computer 10 and the computer 30 are computers that are provided with substantially the same configuration. The computer 10 includes a controller 11 and a storage 12. The computer 30 includes a controller 31 and a storage 32. The controller 11 and the controller 31 are each implemented by a processor such as a central processing unit (CPU). The storage 12 and the storage 32 are each implemented by a solid-state drive, a hard disk drive, or the like.

Hereinafter, further description will be given with the computer 30 taken as an example. The controller 31 executes: a step (1) of selecting a first mode of obtaining backup information 50 corresponding to information stored in the computer 10 which is connected to the analyzer 40 and which controls the analyzer 40, or a second mode of controlling the analyzer 40; a step (2) of obtaining the backup information 50 from the computer 10 if the first mode is selected; and a step (3) of controlling the analyzer 40 if the second mode is selected.

The storage 32 of the computer 30 stores therein: a control program 60 which causes the controller 31 to function as a mode selection part 31a, an analyzer control part 31b, an analysis part 31c, a backup information transmission part 31d, and a backup information reception part 31e; and the backup information 50. The control program 60 is installed in the computer 30. The backup information 50 is a copy of information stored in the storage 12 of the computer 10. The backup information 50 includes: a control program 60c which is a copy of a control program 60 installed in the computer 10; setting information 51c which is a copy of setting information 51 about the control program 60; communication setting information 52c which is a copy of setting information 52 about communication between the computer 10 and the computer 20; and an analysis result 55c which is a copy of an analysis result 55. The backup information 50 does not need to include all of these pieces of information and may include, for example, only the setting information 51c and the communication setting information 52c.

The control program 60 is a program for: controlling of the analyzer 40; communication with the host computer 20; and obtaining of the backup information 50. The controller 31 executes the above steps (1) to (3) by executing the control program 60 installed in the computer 30.

That is, the controller 31 executes the control program 60, to function as the mode selection part 31a, the analyzer control part 31b, the analysis part 31c, the backup information transmission part 31d, and the backup information reception part 31e.

The mode selection part 31a selects, out of a first mode and a second mode, a mode in which the computer 30 is to be operated. The mode selection part 31a selects either the first mode or the second mode on the basis of an operation input received through an input device.

### <First Mode>

If the first mode is selected, the controller 31 functions as the backup information reception part 31e.

The backup information reception part 31e receives the backup information 50 transmitted from the computer 10 and stores the backup information 50 in the storage 32.

The backup information reception part 31e obtains the backup information 50 from the computer 10 via a dedicated communication line 285. Consequently, a communication speed at which the computer 10 communicates with the computer 20 and the analyzer 40, can be inhibited from decreasing. In particular, in a case where the backup information 50 includes information about a scattergram described later, the communication volume for the backup information 50 is large. Thus, if a communication line between the computer 10 and the computer 30 is used also as a communication line between the computer 10 and the computer 20 and/or the analyzer 40, the communication speed at which the computer 10 communicates with the computer 20 and the analyzer 40, decreases. However, if the dedicated communication line 285 is used, such decrease in the communication speed can be inhibited. Although the dedicated communication line is preferably a communication cable that directly connects the computer 30 and the computer 10 to each other, the computer 30 and the computer 10 may be connected to each other via a line concentrator. The communication cable is, for example, a LAN cable. The LAN cable may be a cross cable or a straight cable.

### <Second Mode>

Hereinafter, the second mode will be described with the computer 10 taken as an example. If the second mode is selected, the controller 11 functions as the analyzer control part 31b, the analysis part 31c, and the backup information transmission part 31d.

The analyzer control part 31b controls the analyzer 40 by using the setting information 51 and the control program 60 installed in the computer 10.

The analyzer control part 31b transmits, to the analyzer 40, an instruction to cause the analyzer 40 to start or end measurement of specimens, an instruction regarding a measurement item for each of the specimens, or the like. The analyzer control part 31b obtains a measurement result of a detection-target component from the analyzer 40.

The analysis part 31c analyzes the measurement result obtained from the analyzer 40, generates an analysis result of the specimen, and stores the analysis result as an analysis result 55 in the storage 12.

In addition, the analysis part 31c executes a step of transmitting, to the computer 20, the analysis result 55 stored in the storage 12.

The backup information transmission part 31d generates backup information 50 by copying the setting information 51, the communication setting information 52, the analysis result 55, and the control program 60, and transmits the generated backup information 50 to the computer 30.

### (Control Method for Specimen Analysis System)

Next, a control method for the above specimen analysis system 100 will be described.

In the control method for the specimen analysis system 100 of the present embodiment, the computer 10 controls the analyzer 40. The computer 10 transmits the analysis result 55 to the computer 20 via the network NW. The computer 10 transmits the backup information 50 to the computer 30. During a normal operation of the computer 10, the computer 30 receives the backup information 50 and stores the backup information 50 in the storage 32. Meanwhile, when, for example, the computer 10 has failed, the computer 30 instead of the computer 10 is connected to the analyzer 40 and the computer 20 via communication cables. The computer 30 controls the analyzer 40 on the basis of the setting information 51c, about the control program 60, which is prestored in the storage 32. The computer 30 transmits the analysis result to the computer 20 on the basis of the communication setting information 52c, about the communication, which is prestored in the storage 32.

Consequently, the time required for work of switching from the computer 10 to the alternative computer 30 can be shortened in the specimen analysis system 100.

As shown in FIG. 1, in the computer 10, the second mode is selected during a normal operation of the computer 10 so that the computer 10 is operated as a main computer. Meanwhile, in the computer 30, the first mode is selected during the normal operation of the computer 10 so that the computer 30 is operated as a backup computer. Consequently, the backup information transmission part 31d of the computer 10 transmits the backup information 50, and the backup information reception part 31e of the computer 30 receives the transmitted backup information 50 and stores the backup information 50 in the storage 32. As a result, the backup information 50 is stored in the storage 32 of the computer 30 so as to be kept in an up-to-date state during the normal operation of the computer 10.

As shown in FIG. 2, when the computer 10 has failed, a user of the specimen analysis system 100 performs connection switching of the cables connected to communication ports of the computer 10, to communication ports of the computer 30. That is, the computer 30 is connected to each of the analyzer 40 and the computer 20. Then, the mode selection part 31a of the computer 30 selects the second mode in response to an operation input from the user. Consequently, the operation mode of the computer 30 is switched from the first mode to the second mode. In addition, the mode selection part 31a copies the setting information 51c and the communication setting information 52c into a folder that is located in the storage 32 and that is suitable for execution of the control program 60. In addition, the mode selection part 31a overwrites the control program 60c over the control program 60, to obtain a control program 60. Then, the mode selection part 31a deletes the control program 60c. Consequently, even if the control program 60 has been updated in the computer 10, a version of the control program 60 of the computer 30 is set to be identical to a version of the control program 60 of the computer 10. Meanwhile, if the version of the control program 60 of the computer 30 is up to date or the control program 60 of the computer 30 is updated by another method, the overwriting of the control program 60c over the control program 60 can be omitted.

Consequently, the controller 31 of the computer 30 of which the mode has been switched to the second mode establishes communication connection with the computer 20 via the network NW on the basis of the communication setting information 52c stored in the storage 32. Thereafter, the analyzer control part 31b controls the analyzer 40 and receives a measurement result from the analyzer 40. The analysis part 31c analyzes the measurement result, generates an analysis result, and stores the analysis result in the storage 32. In addition, the analysis part 31c transmits the analysis result to the computer 20 via the network NW

After the computer 30 starts an operation in the second mode, a new backup computer can be introduced to the specimen analysis system 100. The user sets the new computer to be operated in the first mode. As a result, the above operations are performed such that: the computer 30 functions as a main computer; and the new computer functions as a backup computer.

After the computer 30 starts an operation in the second mode, a new main computer may be introduced to the specimen analysis system 100. In this case, the new computer is set to be operated in the second mode. A user performs connection switching of the communication cables connected to the communication ports of the computer 30, to communication ports of the new computer. In addition, the user connects the computer 30 and the new computer to each other via a communication cable. The user sets the computer 30 to be operated in the first mode. As a result, the above operations are performed such that: the new computer functions as a main computer; and the computer 30 functions as a backup computer.

Thus, the present embodiment is as follows. That is, while the computer 10 which controls the analyzer 40 is normally functioning, the computer 30 is operated in the first mode to obtain the backup information 50. Meanwhile, when the computer 10 has failed, the mode of the computer 30 is changed to the second mode so that the analyzer 40 can be controlled by using the prestored backup information 50. Consequently, the time required for work of switching from the other computer 10, which has failed, to the alternative computer 30 can be shortened in the specimen analysis system 100. In particular, expert knowledge about computer networks is required for communication setting of computers. Users of the specimen analysis system 100, in many cases, do not have expert knowledge about computer networks. Thus, for setting of the conventional backup computers, a long time is required, or a user has to wait for a specialized worker to arrive. However, in the present embodiment, the communication setting information 52c is prestored in the computer 30, and thus the switching work can be completed in a short time by changing the mode to the second mode.

### [Configuration of Specimen Analysis System]

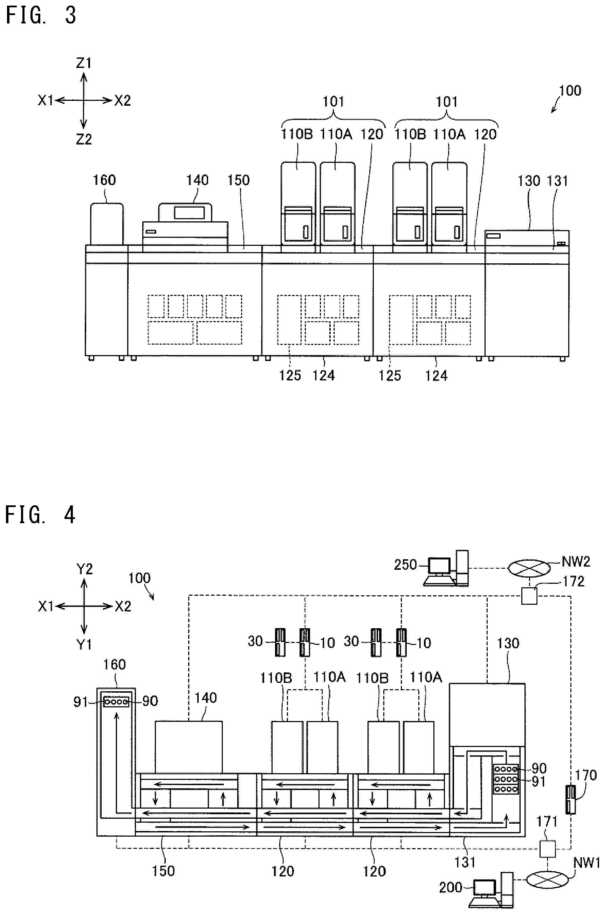

Next, the configuration of the specimen analysis system 100 will be described in detail with reference to FIG. 3 to FIG. 8.

In FIG. 3 and FIG. 4, two directions that are orthogonal to each other on a horizontal plane are defined as an X direction and a Y direction, and an up-down direction is defined as a Z direction. In the Y direction, a frontward direction and a rearward direction of the specimen analysis system 100 are respectively defined as a Y1 direction and a Y2 direction. In the X direction, a leftward direction and a rightward direction of the specimen analysis system 100 are respectively defined as an X1 direction and an X2 direction. In the Z direction, an upward direction and a downward direction of the specimen analysis system 100 are respectively defined as a Z 1 direction and a Z2 direction.

The specimen analysis system 100 shown in FIG. 3 and FIG. 4 includes: first measurement units 110A and second measurement units 110B which are analyzers; transport units 120 which are transporters; the computers 10 (see FIG. 4) which control the measurement units; and the backup computers 30 (see FIG. 4) communicably connected to the computers 10. The first measurement units 110A and the second measurement units 110B are each an example of the analyzer 40.

In the present embodiment, the specimen analysis system 100 includes two unit systems 101 each including: the first measurement unit 110A; the second measurement unit 110B; the transport unit 120; the computer 10; and the backup computer 30. The two unit systems 101 are disposed to be adjacent to each other, and the respective transport units 120 are connected to each other. Each unit system 101 (see FIG. 3) is also a minimum constituent unit of the specimen analysis system 100. The phrase "transport units are connected to each other" means that transport paths are coupled to each other such that a specimen rack 91 can be mutually transferred between the connected transport units.

As shown in FIG. 4, the first measurement unit 110A and the second measurement unit 110B are analyzers for analyzing specimens derived from organisms and are disposed side by side along the transport units 120. The first measurement unit 110A and the second measurement unit 110B are, for example, each a blood analyzer, and specifically, a blood cell counter.

The transport units 120 are disposed frontward (in the Y1 direction) of the measurement units. Each of the transport units 120 is controlled by a transport controller 170. The transport unit 120 includes a plurality of rack transport paths through which the specimen rack 91 can be supplied, in a sorted manner, to either of the first measurement unit 110A and the second measurement unit 110B. The specimen rack 91 can hold a plurality of specimen containers 90 in an upright state. Each of the specimen containers 90 is a container that contains a blood specimen.

In the example in FIG. 4, the specimen analysis system 100 further includes a placement unit 130 in addition to the two unit systems 101. The placement unit 130 is disposed on the upstream side (X2 side) relative to the two unit systems 101. The placement unit 130 is disposed to be adjacent to one unit system 101 that is disposed closer to the upstream side out of the two unit systems 101. The placement unit 130 includes a transport unit 131 which transports the specimen rack 91 to the unit systems 101. The specimen rack 91 holding the specimen containers 90 which have not yet been measured, is set on the transport unit 131 by a user. The transport unit 131 is connected to the transport unit 120 of the adjacent unit system 101.

The specimen analysis system 100 further includes a processing unit 140, a transport unit 150, and a collection unit 160. The processing unit 140 is a device that makes a smear preparation from a blood specimen. The collection unit 160 is a device that collects a specimen container 90 (specimen rack 91) that has been used. The processing unit 140 is disposed to be adjacent to one unit system 101 that is disposed closer to the downstream side (X1 side) out of the two unit systems 101. The collection unit 160 is disposed to be adjacent to the processing unit 140, on the downstream side of the specimen analysis system 100 relative to the processing unit 140.

The transport unit 150 includes a rack transport path through which the specimen rack 91 is transported to the processing unit 140. The transport unit 150 is disposed frontward (in the Y1 direction) of the processing unit 140. The transport unit 150 is connected to each of the transport unit 120 of the unit system 101 and the collection unit 160.

In this manner, in the specimen analysis system 100 in FIG. 4, the placement unit 130, the unit system 101 on the upstream side, the unit system 101 on the downstream side, the processing unit 140, and the collection unit 160 are disposed side by side in this order from the upstream side (X2 side) toward the downstream side (X1 side) in a transport direction of the specimen rack 91. The transport units (120, 131, and 150) of the placement unit 130, the unit system 101 on the upstream side, the unit system 101 on the downstream side, the processing unit 140, and the collection unit 160 are mutually connected to adjacent ones of the transport units. Consequently, the specimen analysis system 100 can sequentially transport the specimen rack 91 disposed in the placement unit 130 to the unit system 101 on the upstream side, the unit system 101 on the downstream side, the processing unit 140, and the collection unit 160 along the transport direction (X1 direction).

As shown in FIG. 3, each of the units of the specimen analysis system 100 is placed on a support. For example, the first measurement unit 110A, the second measurement unit 110B, and the transport unit 120 which compose one of the unit systems 101, are placed on one support 124. A reagent container 125 which contains a reagent to be used by the measurement units is accommodated inside the support 124. The controller 11 of the computer 10 manages information about the type of the reagent, the remaining amount of the reagent, a lot number of the reagent, and an expiration date of the reagent.

As shown in FIG. 4, in the specimen analysis system 100, the computers 10 are connected to a quality control server 200 via a network NW1. The quality control server 200 is an example of the "computer 20". If a quality control specimen containing a predetermined measurement-target component in a known concentration is measured by any of the measurement units, the corresponding computer 10 transmits an analysis result of the quality control specimen to the quality control server 200. The quality control server 200 stores therein the analysis result, of the quality control specimen, which has been transmitted from the computer 10. The network NW1 is, for example, the Internet.

In the specimen analysis system 100, each of the computers 10 is connected to a host computer 250 via a network NW2. The host computer 250 is an example of the "computer 20". The specimen analysis system 100 is disposed in, for example, an examination room in a hospital or the like. In this case, an example of the host computer 250 is a computer that is connected to a plurality of examination instruments and that provides information necessary for each of the examination instruments. This example implements a laboratory information system (LIS). Information about examinations that are performed on specimen containers 90 is registered in the host computer 250. The computer 10 obtains, on the basis of the specimen ID provided on each of the specimen containers 90, a measurement order for the specimen from the host computer 250. If an analysis result of the specimen is generated according to the measurement order, the computer 10 transmits, together with the specimen ID, the generated analysis result to the host computer 250. The host computer 250 stores therein the analysis result transmitted from the computer 10, such that the analysis result is associated with the specimen ID. The network NW2 is, for example, a local area network (LAN) of a facility in which the computer 10 and the host computer 250 are disposed.

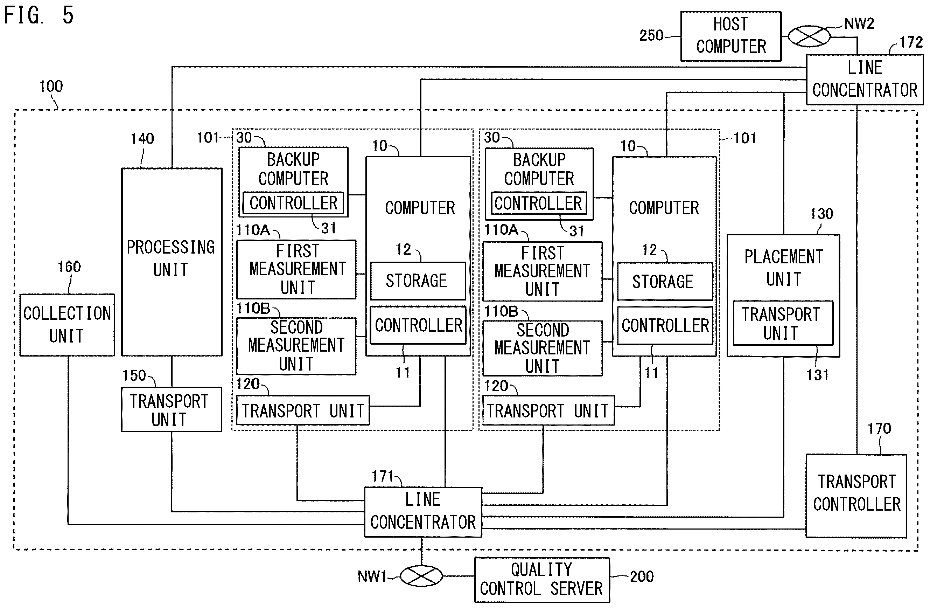

FIG. 5 is a block diagram showing the mutual connection relationship between the units which compose the specimen analysis system 100. As shown in FIG. 5, the computer 10 is provided to each of the unit systems 101. The computer 10 is communicably connected to the measurement units in the same unit system 101 and controls the measurement units in the same unit system 101. The computer 10 controls, by the controller 11 thereof, the first measurement unit 110A and the second measurement unit 110B. In addition, the computer 10 is configured to, by the controller 11 thereof, receive measurement data of a specimen from each of the first measurement unit 110A and the second measurement unit 110B, generate an analysis result of the specimen correspondingly to a measurement item, and store the analysis result in the storage 12.

### (Configuration of Measurement Unit)

The first measurement unit 110A and the second measurement unit 110B are substantially the same type of analyzer. Specifically, the second measurement unit 110B measures a specimen regarding a measurement item that is the same as a measurement item for the first measurement unit 110A, by employing a measurement principle that is the same as a measurement principle employed by the first measurement unit 110A. Further, the second measurement unit 110B performs measurement also regarding a measurement item regarding which no analysis is performed by the first measurement unit 110A. The first measurement unit 110A and the second measurement unit 110B have the same device configuration, and thus the configuration of the first measurement unit 110A will be representatively described below.

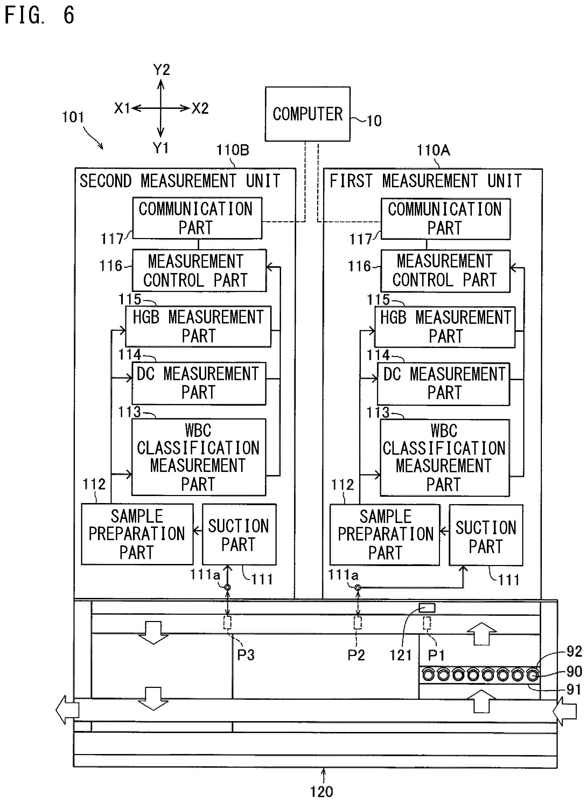

As shown in FIG. 6, the first measurement unit 110A includes a suction part 111, a sample preparation part 112, a WBC classification measurement part 113, a DC measurement part 114, an HGB measurement part 115, a measurement control part 116, and a communication part 117.

The transport unit 120 includes an information reading part 121 which reads a specimen ID from an information storage medium 92 provided on a specimen container 90. The transport unit 120 sequentially transports each of specimen containers 90 held by a specimen rack 91 to a reading position P1 at which reading is to be performed by the information reading part 121. The information reading part 121 reads a specimen ID from the information storage medium 92 on the specimen container 90 disposed at the reading position P1. The information storage medium 92 is, for example, a barcode label having a coded specimen ID printed thereon. The information reading part 121 is, for example, a barcode reader. After the reading of the information, the transport unit 120 transports, to a specimen suction position P2 and/or a specimen suction position P3, the specimen container 90 that is held by the specimen rack 91 and that is to be measured.

The suction part 111 includes: a pipette 111a which suctions the specimen; and a movement mechanism for the pipette 111a. The suction part 111 causes the pipette 111a to move into the specimen container 90 disposed at the specimen suction position P2 and to suction the specimen therein. After the suction of the specimen, the pipette 111a is withdrawn to the outside of the specimen container 90. In a suction part 111 of the second measurement unit 110B, the specimen is suctioned from the specimen container 90 disposed at the specimen suction position P3.

The sample preparation part 112 mixes the specimen and predetermined reagents with each other, to prepare measurement samples that are suitable for measurement processes to be performed by the respective measurement parts. A plurality of types of reagents are used. The reagents include: the reagent in the reagent container 125 disposed inside the support 124; and furthermore, a reagent contained in a reagent container that is set inside the measurement unit.

The WBC classification measurement part 113 is an optical flow cytometer and performs classification and detection of white blood cells by flow cytometry using semiconductor laser light. The WBC classification measurement part 113 detects, with photodiodes, forward scattered light, side scattered light, and side fluorescence which have been outputted from a flow of the measurement sample in association with emission of laser light. The WBC classification measurement part 113 amplifies electrical signals (a forward scattered light signal, a side scattered light signal, and a side fluorescence signal) outputted from the plurality of photodiodes according to the amounts of the received lights. The WBC classification measurement part 113 outputs the amplified electrical signals to the measurement control part 116.

The DC measurement part 114 measures a red blood cell count (RBC) and a platelet count (PLT) by a sheath flow DC detection method. The DC measurement part 114 forms a sample flow enveloped by a sheath flow. The DC measurement part 114 measures a change in the electrical resistance of a detector when the sample flow is caused to pass through an aperture (opening) of the detector. The DC measurement part 114 outputs a measurement result to the measurement control part 116. In addition, the DC measurement part 114 is configured to be capable of also obtaining measurement data for calculating a hematocrit value (HCT) by a red-blood-cell pulse height detection method.

The HGB measurement part 115 measures the amount of hemoglobin (HGB) by an SLS-hemoglobin method. The HGB measurement part 115 emits light to a measurement sample obtained by mixing the specimen, a diluent, and a hemolytic agent. The HGB measurement part 115 measures the absorbance of the measurement sample. The HGB measurement part 115 outputs a measurement result to the measurement control part 116. In the HGB measurement part 115, the concentration of SLS-hemoglobin is measured as an absorbance.

The measurement control part 116 is composed of a central processing unit (CPU), a read-only memory (ROM), a random access memory (RAM), and the like, and controls an operation to be performed by each of the parts of the measurement unit. The communication part 117 is communicably connected to the computer 10. The communication part 117 is, for example, a USB interface. The measurement control part 116 transmits measurement result data to the computer 10 via the communication part 117. In addition, the measurement control part 116 obtains, via the communication part 117, the specimen ID read by the information reading part 121. The measurement control part 116 transmits the specimen ID to the computer 10.

The controller 11 of the computer 10 uses the specimen ID to obtain a measurement item, for the specimen, that corresponds to the specimen ID from the host computer 250. The controller 11 transmits the obtained measurement item to the measurement control part 116. The measurement control part 116 causes, according to content of the received measurement item, any or all of the WBC classification measurement part 113, the DC measurement part 114, and the HGB measurement part 115 to perform measurement operations. The measurement control part 116 transmits, to the computer 10, data of a measurement result corresponding to the measurement item. The controller 11 of the computer 10 generates an analysis result by analyzing the data of the measurement result. The analysis result includes a concentration value of a detection-target component for each measurement item.

### (Hardware Configuration of Computer)

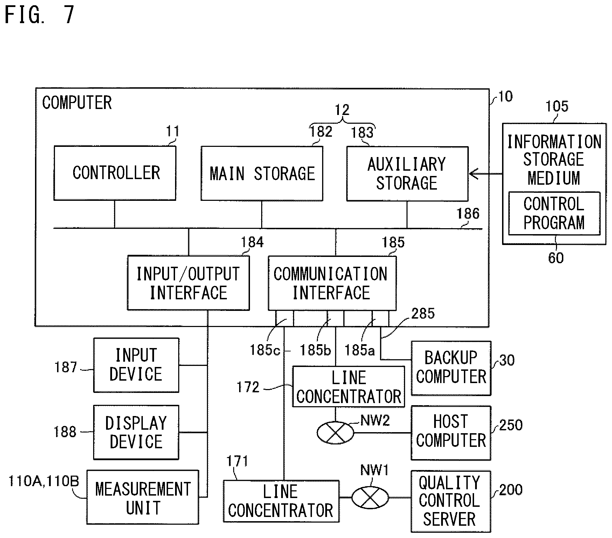

FIG. 7 is a block diagram exemplifying the hardware configuration of the computer 10. The hardware configuration of the computer 30 is the same as that of the computer 10, and thus description will be given with the computer 10 taken as an example. The computer 10 includes the controller 11, a main storage 182, an auxiliary storage 183, an input/output interface 184, and a communication interface 185 which are connected to each other via a bus 186. The controller 11, the main storage 182, and the auxiliary storage 183 are, for example, a CPU, a RAM, and a hard disk drive, respectively. The main storage 182 and the auxiliary storage 183 are each an example of the storage 12.

The auxiliary storage 183 stores therein: an operating system (OS); various types of programs such as the control program 60; the setting information 51 about the control program 60; the communication setting information 52 about the communication; and the like. The control program 60 may be provided by being downloaded from outside of the computer 10 via a network or may be provided from a computer-readable nonvolatile information storage medium 105 such as a semiconductor memory, a CD-ROM, or a DVD-ROM. The control program 60 is installed in the computer 10 and stored in the auxiliary storage 183. The controller 11 develops, onto the main storage 182, the control program 60 stored in the auxiliary storage 183 and executes a command included in the program.

The input/output interface 184 connects the computer 10 and peripheral devices for the computer to each other. The input/output interface 184 is, for example, a USB interface. Peripheral devices such as input devices 187 through which a user inputs various types of information to the computer 10, a display device 188 on which the computer 10 displays the various types of information, and the measurement units (the first measurement unit 110A and the second measurement unit 110B), are connected to the input/output interface 184. The input devices 187 are, for example, a keyboard and a mouse. The display device 188 is, for example, a liquid crystal display. In the example in FIG. 7, the input devices 187, the display device 188, and each of the measurement units are connected to the input/output interface 184 via USB cables.

The communication interface 185 is implemented by a network interface card (NIC) that allows the computer 10 to communicate with an external device. Although one NIC is preferably provided to each communication port, one NIC may be provided to a plurality of communication ports.

The communication interface 185 enables wire communication and wireless communication. The communication interface 185 has a plurality of local area network (LAN) ports (terminals) for wire communication. The LAN ports are connection sockets for so-called LAN cables. In the present embodiment, the communication interface 185 has three or more LAN ports.

A first LAN port 185a is used for communication with the backup computer. The computer 10 and the backup computer 30 are, at respective first LAN ports 185a thereof, connected to each other by a LAN cable without any line concentrator therebetween. Consequently, the backup computer 30 and the computer 10 form a one-to-one network.

A second LAN port 185b is used for communication with the host computer 250. The computer 10 is connected to a line concentrator 172 by a LAN cable connected to the second LAN port 185b, and the line concentrator 172 is connected to the network NW2. The line concentrator is a collective term for one relay device or a plurality of relay devices such as a network hub and a router. The computer 10 can communicate with the host computer 250 via the network NW2. A second LAN port 185b of the backup computer 30 is unused, i.e., unconnected.

A third LAN port 185c is used for communication with the quality control server 200. The computer 10 is connected to a line concentrator 171 by a LAN cable connected to the third LAN port 185c, and the line concentrator 171 is connected to the network NW1. The computer 10 can communicate with the quality control server 200 via the network NW1. A third LAN port 185c of the backup computer 30 is unused, i.e., unconnected.

Static IP addresses different from one another are assigned to these first to third LAN ports. Thus, when the computer 10 has failed, the LAN cables respectively connected to the second LAN port 185b and the third LAN port 185c of the computer 10 are subjected, by a user, to connection switching to equivalent ports (the second LAN port 185b and the third LAN port 185c) of the backup computer 30. As a result, when the backup computer 30 applies the communication setting information 52c, the backup computer 30 can communicate with each of the host computer 250 and the quality control server 200 by using the same static IP addresses as those of the original computer 10. That is, setting change and the like are unnecessary in the case of performing communication from the host computer 250 side and the quality control server 200 side.

### (Functions of Computer 10)

Hereinafter, functions of the computer 10 as a main computer will be described. As shown in FIG. 5, the controller 11 of the computer 10 transmits a generated analysis result to the host computer 250 or the quality control server 200 on the basis of the communication setting information 52. The transmitted analysis result is an analysis result of a specimen or an analysis result of a quality control specimen. The analysis result of the specimen is transmitted to the host computer 250, and the analysis result of the quality control specimen is transmitted to the quality control server 200.

The controller 11 of the computer 10 stores, in the storage 12, information about a measurement result obtained from each measurement unit. The information about the measurement result includes: an identification number of the specimen; information (analysis result) about the concentration of a detection-target component for each measurement item; and information about the patient from whom the specimen has been obtained. These pieces of information about the measurement result are stored so as to be associated with the specimen ID (that is, the identification number of the specimen).

The controller 11 further stores, in the storage 12, information about maintenance of the measurement unit. The information about maintenance of the measurement unit includes: information about various types of histories of errors, alerts, and the like; and information about calibration and quality control. The controller 11 further stores information about instructions for use of the measurement unit. The information about instructions for use is data of a manual.

The controller 11 further stores, in the storage 12, the setting information 51 about the control program 60 for controlling the measurement unit. The setting information 51 about the control program 60 includes: information about a user; information about an evaluation criterion for a measurement result; the number of the analyzers (measurement units) that are connected; an identification number of each of the analyzers (measurement units); and information about an item capable of being measured by the analyzer (measurement unit).

The information about the user is identification information for identifying a user who uses the control program 60. The information about the evaluation criterion for the measurement result is information about a threshold of an abnormal value for each measurement item and is information for determining whether the measurement result is normal (or negative) or abnormal (or positive). The number of the analyzers that are connected is the number of measurement units that are connected to the computer 10. In the example in FIG. 5, the number is two. The identification number of each of the analyzers 40 is information for distinguishing the first measurement unit 110A and the second measurement unit 110B from each other. The information about the item capable of being measured by the analyzer 40 is information about a measurement item capable of being measured in each of the first measurement unit 110A and the second measurement unit 110B. The setting information 51 includes, besides the above pieces of information, information about layout setting of a user interface for the control program 60.

The controller 11 stores the communication setting information 52 about communication with the host computer 250 and the quality control server 200. The communication setting information 52 specifically includes: a static IP address of the host computer 250; information about the static IP addresses, subnet masks, and default gateways of the LAN ports 185a to 185c of the computer 10; and information about DNS setting that is necessary for connection to the quality control server 200.

The controller 11 generates backup information 50 and transmits the generated backup information 50 to the backup computer 30.

The controller 11 transmits the backup information 50 to the backup computer 30 in response to an instruction from a user or according to a schedule specified in advance by a user. The backup computer 30 stores therein the backup information 50 received from the computer 10.

The controller 31 is preferably configured to be operated according to the same type of OS as that of the controller 11.

In the present embodiment, the version of the control program 60 of the backup computer 30 is set to be identical to the version of the control program 60 of the computer 10. Consequently, a malfunction due to the difference in version between the control programs 60 can be prevented from occurring.

Specifically, if the computer 10 applies an update program to upgrade the version of the control program 60, the computer 10 transmits a copy of the update program to the backup computer 30, and the backup computer 30 stores therein the copy of the update program. Then, the backup computer 30 executes the stored copy of the update program. Thus, the control program 60 of the backup computer 30 is updated, and the version thereof becomes identical to the version of the control program 60 of the computer 10.

Consequently, the backup computer 30 can execute the control program 60 in the same version environment as that of the control program 60 of the computer 10. Therefore, a malfunction due to the difference in version between the programs can be prevented.

Alternatively, the version of the control program 60 of the backup computer 30 may be set to be identical to the version of the control program 60 of the computer 10, not by applying the update program to the control program 60, but by rewriting the control program 60 with the control program 60c which is included in the backup information 50 and which is a copy of the control program 60. Alternatively, the control program 60 of the backup computer 30 may be updated by obtaining an update program from an update-program-holding Web server and applying the update program to the control program 60.

### (Backup Information)

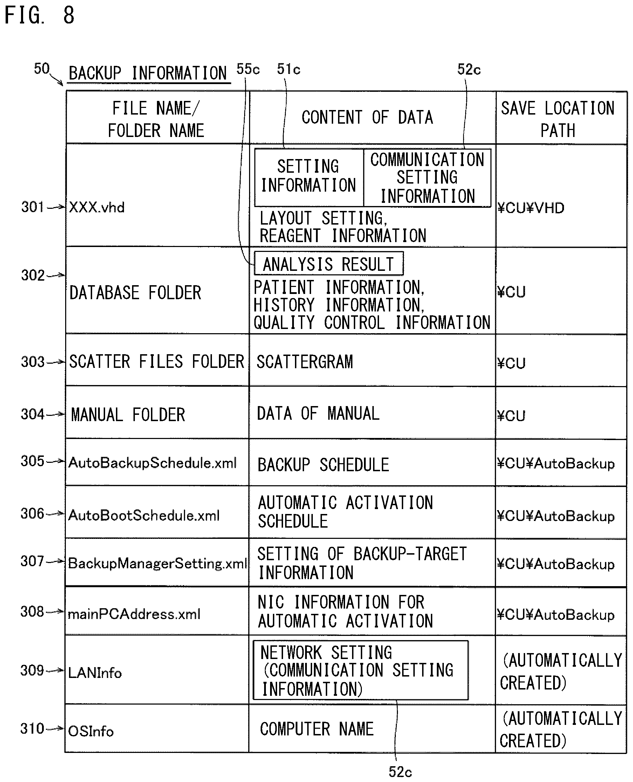

The backup information 50 transmitted to the backup computer 30 includes various types of information shown in FIG. 8.

FIG. 8 shows: file names or folder names of backup-target information; content of data stored in each of the files or the folders; and a save directory (path) of each of the files or the folders in the storage 32 (auxiliary storage 183).

In the present embodiment, the backup computer 30 stores therein the information transmitted from the computer 10, such that a directory structure (the save location paths in FIG. 8) in the computer 10 is maintained. Consequently, it becomes unnecessary to rebuild a directory structure in the backup computer 30.

A"XXX.vhd" file 301 includes: the setting information 51c about the control program 60; the communication setting information 52c about communication with the host computer 250; reagent information about a reagent; and layout setting regarding a screen display layout of the control program 60.

As described above, the setting information 51c includes: information about a user; information about an evaluation criterion for a measurement result; the number of the analyzers (measurement units) that are connected; an identification number of each of the analyzers (measurement units); and information about an item capable of being measured by the analyzer (measurement unit). Consequently, also when the computer 10 has failed, the information about the user, the information about the evaluation criterion for the measurement result, the number of the analyzers 40 that are connected, the identification number of each of the analyzers 40, and the information about the item capable of being measured by the analyzer 40, are handed over to the backup computer 30, whereby the specimen analysis system 100 can be swiftly restored.

The communication setting information 52c about communication with the host computer 250 includes the static IP address of the host computer 250. Consequently, also when the computer 10 has failed, the static IP address of the host computer 250 is handed over to the backup computer 30, whereby the specimen analysis system 100 can be swiftly restored.

A "DATABASE" folder 302 stores therein: an analysis result 55c; patient information about the patient associated with the analysis result; history information indicating a history of an error having occurred; and information about quality control of the measurement unit. The analysis result 55c includes: an identification number of the specimen; and information (analysis result) about the concentration of a detection-target component for each measurement item.

In this manner, the backup information 50 includes the analysis result 55c. In the present embodiment, the computer 10 transmits the analysis result 55c to the backup computer 30, and the backup computer 30 stores therein the analysis result 55c transmitted from the computer 10. Consequently, all data of the analysis result can be backed up in the backup computer 30. Analysis results to be transmitted from the computer 10 to the host computer 250 may be limited to analysis results having values, the accuracies of which have been verified, or may otherwise be only some out of all the analysis results obtained in the computer 10. With this taken into account, loss of an analysis result due to a failure of the computer 10 can be prevented.

In addition, the backup information 50 in the present embodiment includes, in the "DATABASE" folder 302, the patient information associated with the analysis result. That is, the computer 10 transmits the patient information associated with the analysis result to the backup computer 30, and the backup computer 30 stores therein the patient information transmitted from the computer 10, such that the patient information is associated with the analysis result. Consequently, the analysis result and the patient information can be backed up in the backup computer 30, with these pieces of information being kept associated with each other.

In addition, the backup information 50 in the present embodiment includes, in the "DATABASE" folder 302, the information about quality control of the measurement unit. That is, the computer 10 transmits the information about quality control of the measurement unit to the backup computer 30, and the backup computer 30 stores therein the information about quality control transmitted from the computer 10. Consequently, the information about quality control of the measurement unit can be handed over and used in the backup computer 30. Therefore, the analysis result of the quality control specimen does not need to be obtained again at the time of restoration by the backup computer 30.

A "SCATTER FILES" folder 303 stores therein information about graph display of the analysis result generated on the basis of the measurement result. The information about graph display of the analysis result is a scattergram (a graph obtained by plotting dots indicating respective cells in the blood onto a two-dimensional or three-dimensional distribution diagram) and a histogram which are generated on the basis of the measurement result obtained by the measurement unit.

In this manner, in the present embodiment, the analysis result transmitted from the computer 10 to the backup computer 30 includes the information about graph display of the said analysis result. Consequently, the graph display of the analysis result such as the scattergram or the histogram can be stored in the backup computer 30 and can be referred to in the backup computer 30.

A "MANUAL" folder 304 stores therein the information about the manual that can be referred to from the control program 60.

In this manner, the backup information 50 in the present embodiment includes the information about how to handle the measurement unit. Therefore, the computer 10 transmits the information about how to handle the measurement unit to the backup computer 30, and the backup computer 30 stores therein the information, about how to handle the measurement unit, which has been transmitted from the computer 10.

An "AutoBackupSchedule.xml" file 305 includes information about a first schedule according to which automatic backup is to be executed.

An "AutoBootSchedule.xml" file 306 includes information about a second schedule according to which the controller 11 is to be automatically activated by the controller 31. The first schedule and the second schedule will be described later.

A "BackupManagerSetting.xml" file 307 includes information about setting of backup-target information. In the present embodiment, each of the pieces of information which are the analysis result 55c, the patient information, and the scattergram out of the backup information 50 shown in FIG. 8 can be individually set as to whether or not to be included in the backup information 50. This setting is stored in the "BackupManagerSetting.xml" file 307. Information that has been set to be excluded from the backup information 50 is not transmitted from the computer 10 to the backup computer 30. Consequently, a user can voluntarily select whether or not to back up the information about the analysis result and the scattergram which have particularly large amounts of data. If these pieces of information are excluded from the backup information 50, the processing time required for the backup process can be effectively shortened.

A "mainPCAddress.xml" file 308 includes information about a network interface card (NIC) necessary for automatically activating the computer. The automatic activation of the computer will be described later.

A "LANInfo" file 309 includes the communication setting information 52c about communication with the host computer 250 and the quality control server 200. Specifically, the "LANInfo" file includes: the static IP addresses, the subnet masks, and the default gateways of the LAN ports 185a to 185c of the computer 10; and the information about DNS setting that is necessary for connection to the quality control server 200.

An "OSInfo" file 310 includes information about a computer name that is set for the computer 10.

### (Operation of Specimen Analysis System)

A process to be executed by the specimen analysis system 100 will be described with reference to FIG. 9.

As an example of a method for using the specimen analysis system 100, an example will be described in which the computer 10 is not in an activated state but the backup computer 30 is in an activated state at the start of work in an examination room in a hospital or the like. Processing in the computer 10 is executed by the controller 11. Processing in the backup computer 30 is executed by the controller 31.

In step S1, the controller 31 performs an automatic activation process on the computer 10. The details of the automatic activation process will be described later.

In step S2, the controller 11 activates the computer 10 in response to an activation command from the controller 31.

After the automatic activation process, the controller 31 shuts down the computer 30 in step S3.

In step S4, the controller 11 automatically activates the control program 60, and thereafter controls each analyzer 40 according to an examination task.

In step S5, the controller 11 performs a backup process. In the backup process, the backup information including the setting information 51c and the communication setting information 52c is transmitted from computer 10 to the backup computer 30. The details of the backup process will be described later. As an example, step S5 is executed at the end of work of one day in an examination room.

In step S6, the controller 31 activates the backup computer 30 in response to an activation signal transmitted from the computer 10 in step S5, and executes a backup process. The details of the backup process will be described later. Thereafter, the controller 31 continues to be in an operating state until step S10.

In this manner, in the present embodiment, the computer 10 executes transmission of the setting information 51c and the communication setting information 52c after the computer 10 causes activation of the backup computer 30 and the backup computer 30 is activated. Consequently, the setting information can be backed up without a user manually activating the backup computer 30.

In step S7, the controller 11 shuts down the computer 10.

In step S8, the controller 31 performs an automatic activation process on the computer 10. The automatic activation process in step S8 is the same as the process in step S1. As an example, step S8 is executed at the start of work of one day in an examination room. In step S9, the controller 11 activates the computer 10 in response to an activation command from the controller 31. The activation process in step S9 is the same as the process in step S2. In step S10, the controller 31 shuts down the backup computer 30. The shutdown process in step S10 is the same as the process in step S3. In step S11, the controller 11 automatically activates the control program 60, and thereafter controls the analyzer 40 according to an examination task. From this step on, the specimen analysis system repeats the same process.

In this manner, in the present embodiment, during execution of an examination task in an examination facility, the computer 10 is activated and the backup computer 30 is not activated. When backup to the backup computer 30 is performed at a predetermined timing such as the end of the examination task, the controller 11 activates the backup computer 30. With such a configuration, power consumption of the backup computer 30 can be reduced, and moreover, the lifespan of the backup computer 30 is elongated and the stability thereof can be improved.

### (Process of Performing Automatic Activation of Computer 30 and Backup to Computer 30)

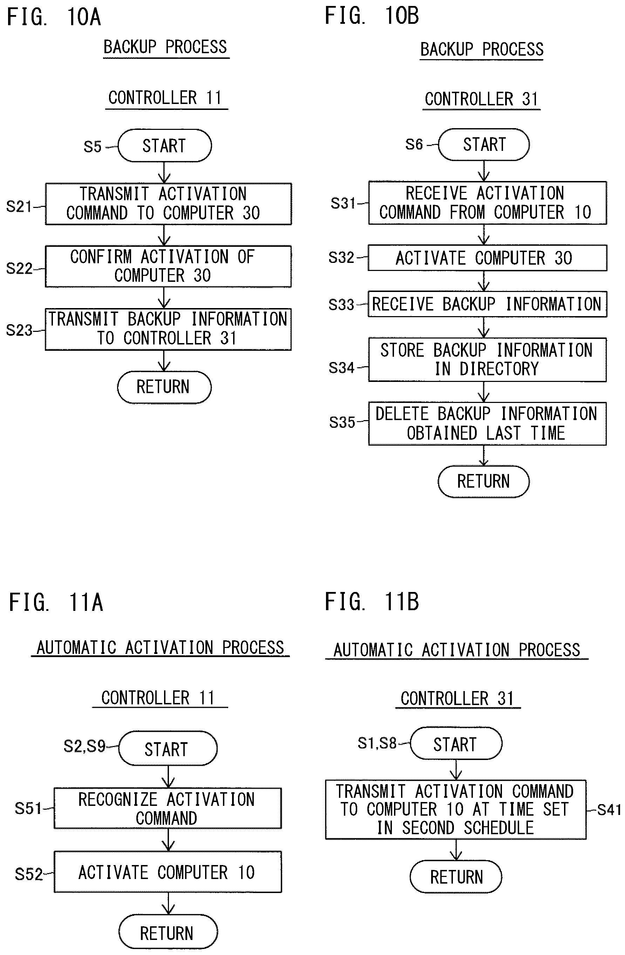

Backup processes to be performed by the controller 11 and the controller 31 will be described with reference to FIG. 10A and FIG. 10B. The backup processes are the processes in step S5 and step S6 in FIG. 9.

The backup process (step S5) to be performed by the controller 11 will be described with reference to a flowchart in FIG. 10A. The controller 11 starts the backup on the basis of: an instruction manually made by a user; or a schedule specified in advance by a user.

In step S21, the controller 11 transmits an activation command to the backup computer 30. The controller 11 activates the backup computer 30 through a wake-on-LAN (WOL) function.

The WOL function is a function of transmitting, by using broadcast transmission based on a TCP/IP protocol, a magic packet to the network interface card (NIC) of a computer that is desired to be activated, to activate the computer. The magic packet is a packet having a payload that includes, at any position in the payload, a data pattern in which the MAC address of a device that is desired to be activated has been repeated 16 times subsequently to FF:FF:FF:FF:FF:FF. That is, the transmitted magic packet is an activation command.

Since the controller 11 uses the WOL function, the controller 11 stores, in the storage 12 of the computer 10, setting information about a TCP/IP program that causes the activation command to be transmitted from the computer 10. The setting information about the TCP/IP program includes NIC information (static IP addresses and subnet masks) and MAC address information about the backup computer 30. In step S21, the controller 11 generates a magic packet on the basis of the stored information about the MAC address and the NIC of the backup computer 30, and transmits the magic packet.

In step S22, the controller 11 confirms that the controller 31 has been activated. The confirmation as to the activation is performed on the basis of: whether or not a shared folder can be accessed; whether or not there is a response from the controller 31 to a response request that uses ping; an activation notification transmitted from the controller 31 to the controller 11; or the like. If the controller 11 confirms that the controller 31 has been activated, the controller 11 advances the process to step S23.

In step S23, the controller 11 transmits the backup information 50 to the backup computer 30. If the controller 31 receives and stores the backup information 50 normally, the controller 31 transmits a completion notification to the computer 10 in response to the transmission of the backup information 50. Meanwhile, if the controller 31 fails to receive and store the backup information 50 normally, the controller 31 transmits an error notification to the computer 10 in response to the transmission of the backup information 50.

If the controller 11 receives the completion notification after the transmission of the backup information 50, the controller 11 shuts down the computer 10 in step S7 in FIG. 9. Meanwhile, if the controller 11 receives the error notification after the transmission of the backup information 50, the controller 11 causes the display device 188 to display the error notification.

Next, the backup process (step S6) to be performed by the controller 31 will be described with reference to the flowchart in FIG. 10B.

In step S31, the controller 31 receives, via the communication interface 185 (network interface card) of the controller 31, the activation command (magic packet) transmitted from the computer 10.

In step S32, the controller 31 activates the backup computer 30 in response to the reception of the activation command via the communication interface 185.

In step S33, the controller 31 receives the backup information 50 transmitted from the controller 11.

In step S34, the controller 31 saves the received backup information 50 in a predetermined directory. That is, the controller 31 stores the backup information 50 in a shared folder of the storage 32 of the controller 31.

As shown in FIG. 8, a script file included in the "XXX.vhd" file 301 specifically defines which information (data file) is to be copied to which directory. The script file is stored in the controller 31 as a part of the backup information 50. The process in step S34 is performed upon execution, by the controller 31, of the script file.

In step S35, the controller 31 deletes, at the time of storing the backup information 50, backup information 50 having been stored during a backup process performed last time. Consequently, a free space in the storage area of the storage 32 can be maintained. Alternatively, the controller 31 may store, in the storage 32, only the difference between the backup information 50 having been stored during the backup process performed last time and the backup information 50 obtained this time. Consequently, the time for the backup process can be shortened.

### (Automatic Activation of Computer 10)

Automatic activation processes for the controller 11 of the computer 10 will be described with reference to FIG. 11A and FIG. 11B. The automatic activation processes are the processes in step S2, S9 and step S1, S8 in FIG. 9.

In the present embodiment, the controller 31 can activate, at a timing specified by a user, the computer 10 which has completed transmission of the backup information and has been shut down. Consequently, the computer 10 can be activated before the start of a task. Thus, specimen measurement by the specimen analysis system 100 can be started immediately after attendance at work.

Firstly, the process (step S1, S8) to be performed by the controller 31 will be described with reference to the flowchart in FIG. 11B.

In step S41, the controller 31 transmits an activation command for activating the computer 10 to the computer 10 on the basis of a schedule that is set in advance.

Since the controller 31 uses the WOL function, the controller 31 stores, in the storage 32, setting information about a TCP/IP program that causes the activation command to be transmitted from the computer 30. The setting information about the TCP/IP program includes NIC information (static IP addresses and subnet masks) and MAC address information about the computer 10. In step S41, the controller 31 generates an activation command (magic packet) on the basis of the stored information about the NIC and the MAC address of the computer 10, and transmits the activation command (magic packet).

As shown in FIG. 9, the controller 31 shuts down the backup computer 30 in steps S3 and S10 after the execution of the automatic activation processes. In this manner, in the present embodiment, the backup computer 30 is automatically turned off after the computer 10 is activated. Consequently, an unnecessary continuous operation of the backup computer 30 is prevented, whereby the power consumption at standby can be reduced. In addition, it is unnecessary for a user to operate the backup computer 30.

Next, the process (step S2, S9) to be performed by the controller 11 will be described with reference to the flowchart in FIG. 11A.

In step S51, the controller 11 recognizes that the communication interface 185 has received the activation command (magic packet) transmitted from the controller 31. As described above, the communication interface 185 is a network interface card (NIC).

In step S52, the controller 11 activates the computer 10.

As shown by steps S4 and S11 in FIG. 9, at the time of the activation, the controller 11 of the computer 10 automatically activates the control program 60 for each measurement unit. Consequently, a user of the computer 10 can start an examination task without performing any operation of activating the control program 60 for the measurement unit.

### (Schedule Function)

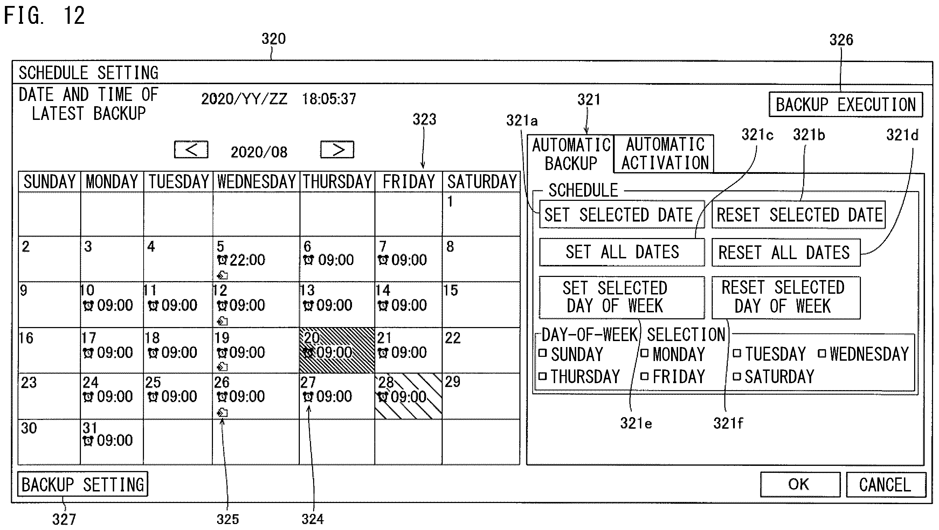

Next, a schedule function in the specimen analysis system 100 will be described with reference to FIG. 12 to FIG. 14.

In the present embodiment, the computer 10 activates the backup computer 30 on the basis of a first schedule that is set in advance. The computer 10 transmits the backup information 50 to the backup computer 30 after the backup computer 30 is activated. Consequently, the process of performing activation of the backup computer 30 and backup to the backup computer 30 can be automatically executed according to the schedule. The first schedule is an execution schedule for the backup process including automatic activation of the computer 30.

In addition, the backup computer 30 activates the computer 10 on the basis of a second schedule that is set in advance. Consequently, it is unnecessary for a user to manually perform operation for the activation, and the computer 10 can be automatically activated according to the schedule by using the backup computer 30 for backup. The second schedule is an execution schedule for automatic activation of the main computer at the start of a task.

In this manner, in the present embodiment, the first schedule according to which the controller 11 of the computer 10 executes the backup process and the second schedule according to which the controller 31 of the computer 30 automatically activates the computer 10 are set in advance, and thus the backup process and the activation process are automatically executed according to the schedules.

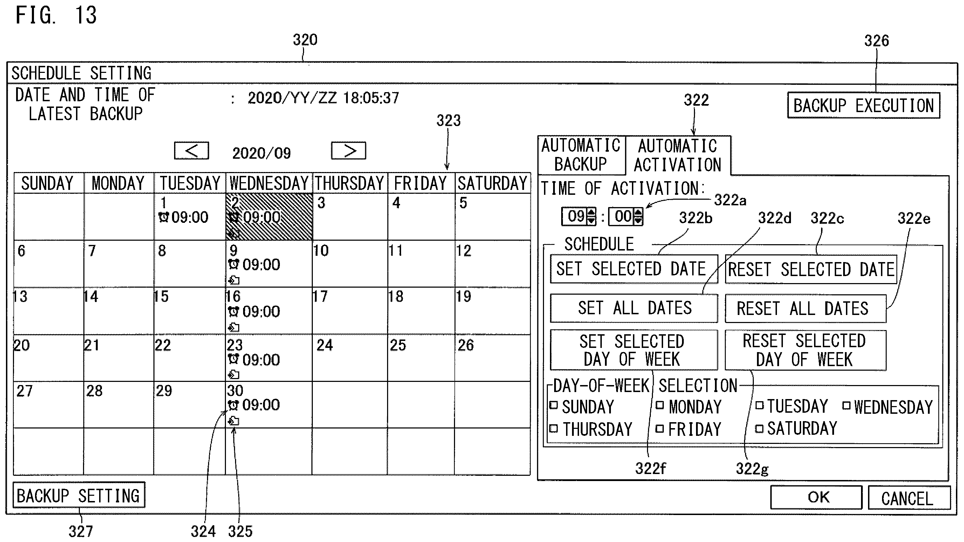

FIG. 12 and FIG. 13 each show a graphical user interface (GUI) 320 which is displayed by executing the control program 60 and which enables setting of both the first schedule and the second schedule by switching between tabs. FIG. 12 shows an example of a first schedule setting screen 321, and FIG. 13 shows an example of a second schedule setting screen 322. These setting screens are displayed on the display device 188 (see FIG. 7) of the computer 10. Through the setting screens, a user performs operation input.

That is, in the present embodiment, the computer 10 receives: the first schedule according to which the backup computer 30 is to be activated; and the second schedule according to which the computer 10 is to be activated. Consequently, setting of the first schedule for the computer 10 and setting of the second schedule for the backup computer 30 can be performed merely by performing an operation on the computer 10 which is a main computer.

Icons 324 indicating that automatic activation schedules (second schedules) have been set, and times of the schedules, are displayed at respective dates in a calendar 323 displayed in common in FIG. 12 and FIG. 13. In addition, icons 325 indicating that automatic backup schedules (first schedules) have been set are displayed at respective dates in the calendar 323. The GUI 320 may be configured to display thereon: information about a date and a time at which the latest backup has been performed; and information indicating that backup has failed.

In the first schedule setting screen 321, setting of a date on which backup is to be executed is received. The first schedule setting screen 321 is provided with: a button 321a for setting, as a backup execution date, a date selected from the calendar 323; a button 321b for canceling the setting, as the backup execution date, of the date selected from the calendar 323; a button 321c for setting, as backup execution dates, all the dates in the calendar 323; a button 321d for canceling the setting, as the backup execution dates, of all the dates in the calendar 323; a button 321e for setting, as backup execution dates, dates corresponding to a day of each week selected from a day-of-week selection field; and a button 321f for canceling the setting, as the backup execution dates, of the dates corresponding to the day of the week selected from the day-of-week selection field. Since the automatic backup process based on the first schedule is executed when a shutdown instruction is received, a time at which the backup process is to be executed is not set.

In the second schedule setting screen 322, setting of a date and a time at which the computer 10 is to be automatically activated from the backup computer 30 is received. The second schedule setting screen 322 is provided with: a setting field 322a for a time of activation; a button 322b for setting, as a specified date for automatic activation, a date selected from the calendar 323; a button 322c for canceling the setting, as the specified date, of the date selected from the calendar 323; a button 322d for setting, as specified dates, all the dates in the calendar 323; a button 322e for canceling the setting, as the specified dates, of all the dates in the calendar 323; a button 322f for setting, as specified dates, dates corresponding to a day of each week selected from a day-of-week selection field; and a button 322g for canceling the setting, as the specified dates, of the dates corresponding to the day of the week selected from the day-of-week selection field. Consequently, the automatic activation process based on the second schedule is executed when a time of activation on a specified date has come.

Regarding each second schedule, the computer 10 receives setting of the second schedule and sets the received second schedule with respect to the backup computer 30. Consequently, it is unnecessary for a user to operate the computer 10 and the backup computer 30 separately, and the setting with respect to the backup computer 30 can be performed merely by performing an operation on the computer 10 which is a main computer. Each second schedule that has been set in the second schedule setting screen 322 is stored in the "AutoBootSchedule.xml" file 306 of the backup information 50 shown in FIG. 8 and is transmitted from the computer 10 to the backup computer 30 at the time of the backup process.

The setting information about the second schedule according to which the computer 10 is to be activated from the computer 30, includes setting information about the TCP/IP program that causes an activation command to be transmitted from the computer 30. Consequently, the activation process for the computer 10 can be performed through the WOL function using TCP/IP which is a standard communication protocol. Thus, neither dedicated lines nor dedicated devices for performing automatic activation need to be provided.

In the GUI 320, a backup execution instruction that is made manually can also be received. That is, the GUI 320 is provided with a backup execution button 326. When the backup execution button 326 is selected, the controller 11 executes the backup process in step S5. In response to the execution, the controller 31 executes the backup process in step S6. In this case, the controller 11 may cause the display device 188 to display thereon, for example, a dialog box for asking whether or not the backup process may be started.

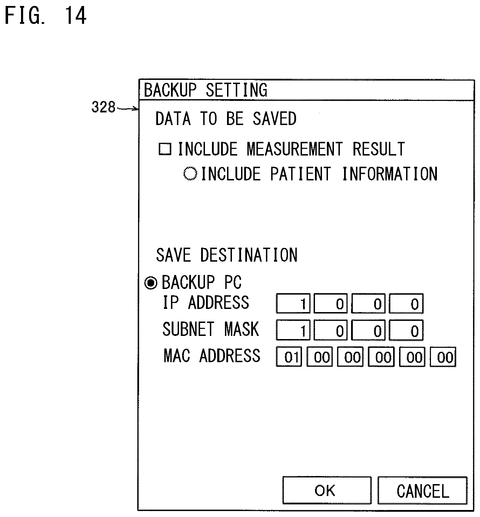

It is also possible to perform input through a backup setting button 327 in the GUI 320, to invoke a setting screen 328 (shown in FIG. 14) for performing setting of backup-target information. As setting about the backup process, setting can be made as to: whether or not an analysis result is to be included as information to be backed up; and, in the case of including the analysis result, whether or not patient information related to the analysis result is also to be included. If selection is made so as not to include the analysis result (if the checkbox is unchecked), the information about the scattergram is also excluded from backup targets. Content of this setting is stored in the "BackupManagerSetting.xml" file 307 of the backup information 50 shown in FIG. 8.

In the setting screen 328, setting information about the TCP/IP program that causes an activation command to be transmitted from the computer 10, can be set. Specifically, the setting information about the TCP/IP program includes information about the static IP addresses, the subnet masks, and the MAC address, of the computer 30, which are for transmitting an activation command to the computer 30. These pieces of information can be set from the setting screen 328.

Each first schedule that has been set in the first schedule setting screen 321 is also included in the backup information 50 which is stored in the backup computer 30. That is, the computer 10 transmits setting information about the first schedule to the backup computer 30, and the backup computer 30 stores therein the setting information about the first schedule. The setting information about the first schedule is stored in the "AutoBackupSchedule.xml" file 305 of the backup information 50 shown in FIG. 8.

The setting information about the first schedule is not used while the backup computer 30 is being operated in the second mode. If the main computer 10 fails and the backup computer 30 is operated as a main computer in the second mode, the computer 30 can hand over, to a newly introduced computer, the setting of the first schedule.

### (Operation at Time of Failure of Computer 10)

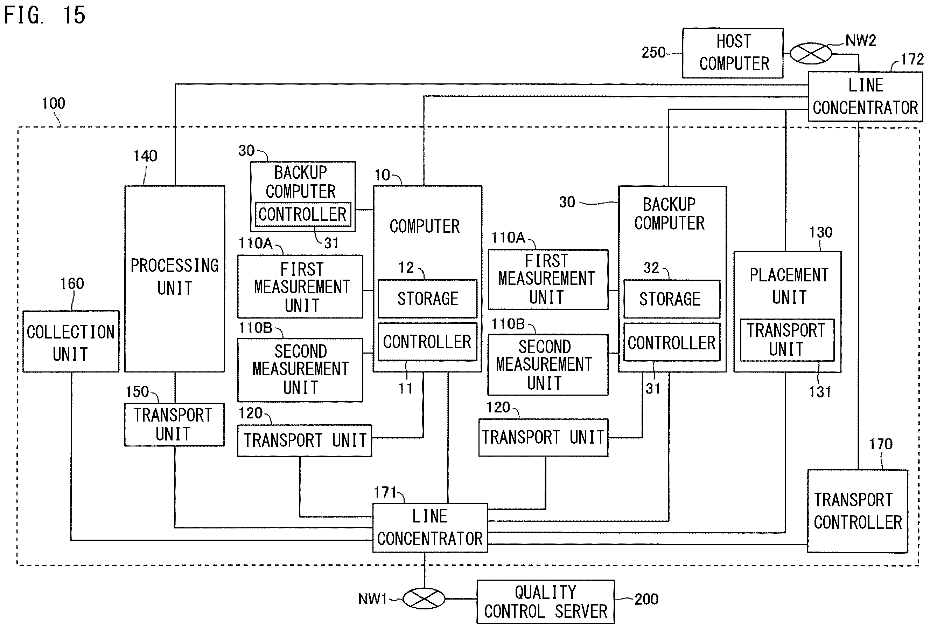

FIG. 15 is a system configuration diagram in a state where: a computer 10 has failed; and the computer 10 and a corresponding computer 30 which is a backup computer have been exchanged with each other.

FIG. 15 shows, as an example, a situation in which one computer 10 out of the two computers 10 has failed, and thus the specimen analysis system 100 is restored by the corresponding computer 30 which is a backup computer. The computer 30 instead of the computer 10 is connected to the first measurement unit 110A, the second measurement unit 110B, the transport unit 120, the host computer 250, and the quality control server 200. As shown in FIG. 2, the control program 60 for controlling each of the measurement units, the setting information 51c about the control program 60, the communication setting information 52c about communication with the quality control server 200 and the host computer 250, and the like are stored in the storage 32 of the computer 30. Thus, the specimen analysis system can be swiftly restored even when a failure of the computer 10 has occurred.

The computer 30 and the host computer 250 are connected to each other at the LAN ports equivalent to those at which the computer 10 and the host computer 250 have been connected to each other. In the same manner, connection between the computer 30 and the transport unit 120 and connection between the computer 30 and the quality control server 200 are also made at the LAN ports equivalent to those of the computer 10. The computer 30 and each of the first measurement unit 110A and the second measurement unit 110B, are connected to each other by a USB cable disconnected from the computer 10.

The backup information 50 stored in the computer 30 may include a program for executing a tutorial showing a connection switching procedure for USB cables and LAN cables at the time of restoration. Consequently, a user can unmistakably perform connection switching from the computer 10, which has failed, to the backup computer 30.

In the present embodiment, when the computer 10 has failed, the computer 30 receives an applying instruction for the backup information 50. The computer 30 applies the setting information 51c about the control program 60 and the communication setting information 52c to the computer 30 on the basis of the received applying instruction. Consequently, a process of applying, to the computer 30, information stored by the computer 30 until the occurrence of the failure of the computer 10, can be performed merely by inputting the applying instruction to the backup computer 30.

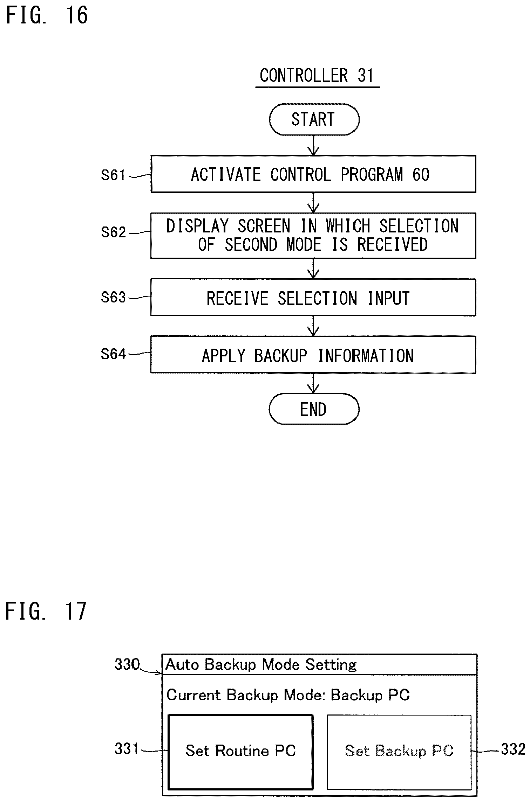

FIG. 16 explains an operation to be performed when the specimen analysis system 100 is restored by using the computer 30.

When a user powers on the computer 30 and completes activation processes for an operating system (OS) and the like, the controller 31 activates the control program 60 in step S61.

In step S62, the controller 31 displays a screen 330 (see FIG. 17) in which selection of the second mode is received in order to use, as a main computer for controlling each measurement unit, the computer 30 having been used as a backup computer up until now.

The screen 330 includes a "Set Routine PC" button 331 and a "Set Backup PC" button 332. The "Set Routine PC" button 331 is a button for inputting an instruction to set the mode of the computer to the second mode. The "Set Backup PC" button 332 is a button for inputting an instruction to set the mode of the computer to the first mode.

As shown in FIG. 17, if the first mode is selected (that is, if the computer 30 is being operated as the backup computer), a step (step S62) of displaying the screen 330 in which selection of the second mode is received, is executed at the time of activation of the computer 30. Consequently, if the computer 10 fails and it becomes necessary to switch the mode of the computer 30 from the first mode to the second mode, operation of switching to the second mode can be performed merely by activating the computer 30. Thus, convenience for a user who is inexperienced in setting work is improved.

In step S63, the controller 31 receives input performed through the "Set Routine PC" button 331 in the screen 330 shown in FIG. 17.

In step S64, the controller 31 performs switching from the first mode to the second mode in response to the input performed through the "Set Routine PC" button 331, to start an operation in the second mode. The controller 31 performs a process of applying the backup information 50.

More specifically, the controller 31 applies, to setting information about the communication interface 185 and an OS, the static IP address of the host computer 250 stored in the "XXX.vhd" file 301. In addition, the controller 31 applies, to the setting information about the communication interface 185 and the OS, the static IP addresses, the subnet masks, and the default gateways of the computer 10 stored in the "LANInfo" file 309 and the DNS setting of the quality control server 200. A method for the applying is performed with the control program 60 and the OS. For example, the method includes storing information such as the static IP addresses and the DNS setting in a folder specified by the control program 60.

When the computer 30 receives the applying instruction, the computer 30 executes a process of applying, to the backup computer 30, the setting information for automatically executing the control program 60 for each measurement unit. Specifically, the controller 31 sets a flag such that the second mode is automatically selected at the time of activation of the computer 30. In addition, the computer 30 executes a process of applying, to the backup computer 30, information about a computer name of the computer 10, the setting information about the first schedule according to which the backup computer 30 is to be activated, and the setting information about the second schedule according to which the computer 10 is to be activated.

Consequently, the applying of the setting information for automatically executing the control program 60 makes it possible to execute a function of controlling the measurement unit instead of the computer 10 merely by activating the backup computer 30. The applying of the information about the computer name of the computer 10 makes it possible to immediately perform, as the computer 10, communication with the computer 20. The applying of the setting information about the first schedule and the applying of the setting information about the second schedule make it possible to, in the case of introducing a new computer as a computer for next backup, continue schedule setting applied before the introduction, without any change.

Specifically, the controller 31 uses the "XXX.vhd" file 301 to apply, as a startup process at the time of activation from the next time on, setting for automatically activating the control program 60 for the measurement unit. In addition, the controller 31 obtains, from the "OSInfo" file 310, the computer name used for the computer 10 which has failed, and newly applies, as a computer name of the computer 30, the computer name to the OS thereof. In addition, the controller 31 uses the "AutoBackupSchedule.xml" file 305 to apply the setting information about the first schedule to the control program 60. In addition, the controller 31 uses the "AutoBootSchedule.xml" file 306 to apply the setting information about the second schedule to the control program 60. When a newly introduced backup computer is connected to the computer 30, the setting information about the second schedule is reflected in the backup computer.

When these settings are completed, the controller 31 reactivates the OS. The controller 31 which has performed the reactivation, starts an operation in the second mode and can be operated in the same manner as the controller 11 of the computer 10 which is a main computer. Connection of the new backup computer to the computer 30 does not need to be immediately executed, and thus a user can execute the connection during inactivity of work or can also ask a field engineer to work on the connection.

If the second mode is selected in the backup computer 30, a step of establishing connection between the computer 30 and the host computer 250 is executed by using the setting information 52c about communication with the host computer 250 and the quality control server 200 after the reactivation. Consequently, when the computer 10 has failed, communication connection between the host computer 250 and the computer 30 as a substitute for the computer 10 can be established merely through changing, by a user, the mode of the computer 30 to the second mode without performing any communication setting work.

In this example, the backup computer 30 executes a process of applying two or more of pieces of information that are further applied to the backup computer 30, by receiving one time of applying instruction (input performed through the "Set Routine PC" button 331) in step S63. That is, the setting information for automatically executing the control program 60 for the measurement unit, the information about the computer name of the computer 10, the setting information about the first schedule according to which the backup computer 30 is to be activated, and the setting information about the second schedule according to which the computer 10 is to be activated, are each applied according to the one time of applying instruction. Consequently, work to be executed by a user is simplified, and thus convenience for a user who is inexperienced in setting operation for computers is improved.

In FIG. 17, the only choice through which input can be performed is one button, i.e., the "Set Routine PC" button 331, and the "Set Backup PC" button 332 is in a grayed-out state in which input cannot be performed through the "Set Backup PC" button 332. That is, FIG. 17 shows that: the computer 30 is currently being operated in the second mode in a state where input has been performed through the "Set Backup PC" button 332; and switching from the second mode to the first mode can be performed by performing input through the " Set Routine PC" button 331. If, for example, the computer 30 is activated during a normal operation of the computer 10, the computer 30 continues to be operated in the second mode without any change unless input is performed through the "Set Routine PC" button 331.

### [Modification]

It should be noted that the embodiment disclosed herein is merely illustrative in all aspects and should not be considered as being restrictive. The scope of the present disclosure is not defined by the description of the above embodiment but by the scope of the claims, and further includes all changes (modifications) within meanings and scopes that are equivalent to the scope of the claims.

For example, although an example in which the specimen analysis system 100 includes two unit systems 101 has been described in the above embodiment, the present disclosure is not limited to this example. The specimen analysis system 100 may include one unit system 101 or may include three or more unit systems 101. Further, although an example in which each unit system 101 includes two measurement units has been described, the present disclosure is not limited to this example. The unit system 101 may include one measurement unit or may include three or more measurement units.

Further, although an example in which one backup computer 30 is provided to each of the two unit systems 101 has been described in the above embodiment, the present disclosure is not limited to this example. In the present disclosure, one backup computer 30 may be provided to the two unit systems 101. In this case, the backup computer 30 stores therein each of: the backup information 50 from the computer 10 of one of the unit systems 101; and the backup information 50 from the computer 10 of the other unit system 101. With this configuration, if the mode of the backup computer 30 is switched to the second mode, selection can be made as to whether the backup information 50 from the computer 10 of the one unit system 101 is to be applied or the backup information 50 from the computer 10 of the other unit system 101 is to be applied.

Further, although an example in which each measurement unit which is an analyzer is a blood cell counter has been described in the above embodiment, the present disclosure is not limited to this example. The analyzer may be a device other than a blood cell counter. The analyzer may be, for example, a nucleic acid analyzer, a cell image analyzer, a blood coagulation measurement device, an immunoassay device, a urine particle measurement device, or the like. The analyzer may also be another analyzer.

Further, although an example in which the specimen analysis system 100 includes the placement unit 130, the processing unit 140 and the transport unit 150, and the collection unit 160 has been described in the above embodiment, the present disclosure is not limited to this example. The specimen analysis system 100 may be provided with no placement unit 130. The specimen rack 91 may be placed in the transport unit 120. The specimen analysis system 100 may be provided with neither processing unit 140 nor transport unit 150. The specimen analysis system 100 may be provided with no collection unit 160.

Further, although an example in which the computer 10 and the line concentrator 171 for connection to the backup computer 30, the host computer 250, and the quality control server 200 are connected to each other in a wired manner, i.e., via LAN cables, has been described in the above embodiment, the present disclosure is not limited to this example. In the present disclosure, the computer 10 and each of the backup computer 30, the host computer 250, and the quality control server 200 may be wirelessly connected to each other. For example, communication connection therebetween can be performed according to a wireless LAN standard such as Wi-Fi (registered trademark).

Further, although the quality control server 200 and the host computer 250 have been described as specific examples of the computer 20 in the above embodiment, the computer 20 in the present disclosure is not limited to these specific examples. In the present disclosure, one or both of the quality control server and the host computer may be excluded. Alternatively, the second computer may be a server or a computer other than the quality control server and the host computer.

Further, although an example in which, when the computer 10 has failed, the backup computer 30 executes the process of further applying, to the backup computer 30, the setting information for automatically executing the control program 60 for each measurement unit, the information about the computer name of the computer 10, the setting information about the first schedule according to which the backup computer 30 is to be activated, and the setting information about the second schedule according to which the computer 10 is to be activated, has been described in the above embodiment, the present disclosure is not limited to this example. In the present disclosure, only some of these pieces of information may be applied to the backup computer 30. Alternatively, the process of applying these pieces of information does not have to be executed.

Further, although an example in which the setting information 51, 51c about the control program 60 includes information about a user, information about an evaluation criterion for a measurement result, the number of the measurement units that are connected, an identification number of each of the measurement units, and information about an item capable of being measured by the measurement unit, has been described in the above embodiment, the present disclosure is not limited to this example. In the present disclosure, the setting information 51, 51c about the control program 60 may include only some of these pieces of information. Alternatively, the setting information 51, 51c about the control program 60 does not have to include these pieces of information.

## Claims

1. A control method for a specimen analysis system comprising an analyzer configured to analyze a specimen, the control method comprising:
controlling, by a first computer, the analyzer;
transmitting, by the first computer, an analysis result to a second computer communicably connected to the first computer via a network and configured to manage the analysis result;
transmitting, by the first computer, setting information about a control program for the analyzer and setting information about communication with the second computer, to a third computer communicably connected to the first computer;
storing, by the third computer, the setting information about the control program and the setting information about the communication which have been transmitted from the first computer; and
controlling, by the third computer instead of the first computer, the analyzer on the basis of the stored setting information about the control program, and transmitting, by the third computer instead of the first computer, an analysis result to the second computer on the basis of the stored setting information about the communication.

2. The control method for the specimen analysis system, of claim 1, the control method further comprising:
receiving, by the third computer, an applying instruction for the stored setting information about the control program and the stored setting information about the communication, wherein
upon the receiving of the applying instruction, the third computer executes the controlling of the analyzer and the transmitting of the analysis result to the second computer.

3. The control method for the specimen analysis system, of claim 2, wherein
the third computer receives the applying instruction through one time of input operation.

4. The control method for the specimen analysis system, of claim 2 or 3, wherein the third computer
displays a screen in which the applying instruction is received, and
receives the applying instruction through an input operation performed via the screen.

5. The control method for the specimen analysis system, of any one of claims 2 to 4, wherein
upon the receiving of the applying instruction, the third computer
applies the setting information about the control program and the setting information about the communication, to the third computer, and
executes the controlling of the analyzer and the transmitting of the analysis result to the second computer.

6. The control method for the specimen analysis system, of claim 5, wherein
upon the receiving of the applying instruction, the third computer executes a process of further applying, to the third computer, at least one of: setting information for automatically executing the control program for the analyzer; information about a computer name of the first computer; setting information about a first schedule according to which the third computer is to be activated; and setting information about a second schedule according to which the first computer is to be activated.

7. The control method for the specimen analysis system, of any one of claims 1 to 6, wherein
the first computer transmits the control program for the analyzer or update information for the control program, to the third computer,
the third computer stores therein the control program or the update information for the control program, and,
when the first computer has failed, the third computer controls the analyzer on the basis of: the control program or a control program obtained by update thereof with the update information; and the setting information about the control program.

8. The control method for the specimen analysis system, of any one of claims 1 to 7, wherein
the first computer activates the third computer, and,
after the third computer is activated, the first computer executes the transmitting of the setting information about the control program and the setting information about the communication.

9. The control method for the specimen analysis system, of claim 8, wherein
the first computer activates the third computer on the basis of a first schedule that is set in advance.

10. The control method for the specimen analysis system, of any one of claims 1 to 9, wherein
the first computer is automatically turned off after the first computer transmits the setting information about the control program and the setting information about the communication, to the third computer.

11. The control method for the specimen analysis system, of claim 10, wherein
the third computer activates the first computer on the basis of a second schedule that is set in advance.

12. The control method for the specimen analysis system, of claim 11, wherein
the first computer receives setting of the second schedule and sets the received second schedule with respect to the third computer.

13. The control method for the specimen analysis system, of claim 12, wherein
the first computer receives: a first schedule according to which the third computer is to be activated; and the second schedule according to which the first computer is to be activated.

14. The control method for the specimen analysis system, of any one of claims 11 to 13, wherein
setting information about the second schedule according to which the first computer is to be activated, comprises setting information about a TCP/IP program that causes an activation command to be transmitted from the third computer.

15. The control method for the specimen analysis system, of any one of claims 9 to 14, wherein
the third computer is automatically turned off after the first computer is activated.

16. The control method for the specimen analysis system, of claim 9, wherein
the first computer transmits setting information about the first schedule to the third computer, and
the third computer stores therein the setting information about the first schedule.

17. The control method for the specimen analysis system, of any one of claims 1 to 16, wherein
the analysis result is an analysis result of the specimen or an analysis result of a quality control specimen.

18. The control method for the specimen analysis system, of any one of claims 1 to 17, wherein
the setting information about the control program comprises at least one of:
information about a user; information about an evaluation criterion for a measurement result; the number of the analyzers that are connected; an identification number of each of the analyzers;
and information about an item capable of being measured by the analyzer.

19. The control method for the specimen analysis system, of any one of claims 1 to 18, wherein
the first computer transmits the analysis result to the third computer, and
the third computer stores therein the analysis result transmitted from the first computer.

20. The control method for the specimen analysis system, of claim 19, wherein
the first computer transmits, to the third computer, information about a patient associated with the analysis result, and
the third computer stores therein the information about the patient transmitted from the first computer, such that the information about the patient is associated with the analysis result.

21. The control method for the specimen analysis system, of claim 19 or 20, wherein
the analysis result transmitted from the first computer to the third computer comprises information about graph display of the analysis result.

22. The control method for the specimen analysis system, of any one of claims 1 to 21, wherein
the third computer stores therein the information transmitted from the first computer, such that a directory structure in the first computer is maintained.

23. The control method for the specimen analysis system, of any one of claims 1 to 22, wherein
the first computer transmits, to the third computer, information about how to handle the analyzer, and
the third computer stores therein the information about how to handle the analyzer, the information having been transmitted from the first computer.

24. The control method for the specimen analysis system, of any one of claims 1 to 23, wherein
the first computer transmits information about quality control of the analyzer to the third computer, and
the third computer stores therein the information about the quality control transmitted from the first computer.

25. The control method for the specimen analysis system, of any one of claims 1 to 24, wherein
a version of the control program of the third computer is set to be identical to a version of the control program of the first computer.

26. The control method for the specimen analysis system, of claim 25, wherein
the first computer transmits, to the third computer, update information for updating the control program of the third computer to a predetermined version, and
the third computer updates the control program installed in the third computer, according to the update information transmitted from the first computer.

27. A specimen analysis system comprising:
an analyzer configured to analyze a specimen;
a first computer configured to control the analyzer and transmit, via a network, an analysis result to a second computer configured to manage the analysis result; and
a third computer communicably connected to the first computer, wherein
the first computer transmits, to the third computer, setting information about a control program for the analyzer and setting information about communication with the second computer,
the third computer stores therein the setting information about the control program and the setting information about the communication which have been transmitted from the first computer, and
the third computer instead of the first computer controls the analyzer on the basis of the stored setting information about the control program and transmits an analysis result to the second computer on the basis of the stored setting information about the communication.

28. A control method for a specimen analysis system, the control method being for executing, by a computer, controlling of an analyzer and obtaining of backup information about the analyzer, the control method comprising:
selecting
a first mode of obtaining the backup information corresponding to information stored in another computer connected to the analyzer, the other computer being configured to control the analyzer, or
a second mode of controlling the analyzer;
obtaining the backup information from the other computer if the first mode is selected; and
controlling the analyzer if the second mode is selected.

29. The control method for the specimen analysis system, of claim 28, wherein
the backup information comprises a control program for the analyzer or setting information about the control program, and
the controlling of the analyzer comprises controlling, after the obtaining of the backup information is executed, the analyzer by using the control program or the setting information if the second mode is selected.

30. The control method for the specimen analysis system, of claim 28 or 29, wherein
if the first mode is selected, the analyzer is controlled by the other computer.

31. The control method for the specimen analysis system, of any one of claims 28 to 30, the control method further comprising
transmitting, if the second mode is selected, second backup information corresponding to information stored in the computer, to a separate computer that is not connected to the analyzer but is connected to the computer.

32. The control method for the specimen analysis system, of any one of claims 28 to 31, wherein
the obtaining of the backup information comprises obtaining the backup information from the other computer via a dedicated communication line.

33. The control method for the specimen analysis system, of any one of claims 28 to 32, wherein
the other computer is connected via a network to a host computer configured to manage an analysis result obtained by the analyzer,
the backup information comprises setting information about communication between the other computer and the host computer, and
the control method further comprises establishing, if the second mode is selected, connection between the computer and the host computer by using the setting information about the communication between the other computer and the host computer.

34. The control method for the specimen analysis system, of any one of claims 28 to 33, the control method further comprising
displaying, if the first mode is selected, a screen in which selection of the second mode is received, the displaying being performed when the computer is activated.

35. A computer configured to execute controlling of an analyzer and obtaining of backup information about the analyzer, the computer comprising
a controller, wherein
the controller executes
selecting
a first mode of obtaining the backup information corresponding to information stored in another computer connected to the analyzer, the other computer being configured to control the analyzer, or
a second mode of controlling the analyzer,
obtaining the backup information from the other computer if the first mode is selected, and
controlling the analyzer if the second mode is selected.

36. A program configured to cause controlling of an analyzer and obtaining of backup information about the analyzer, the program being configured to cause a computer to execute:
selecting
a first mode of obtaining the backup information corresponding to information stored in another computer connected to the analyzer, the other computer being configured to control the analyzer, or
a second mode of controlling the analyzer;
obtaining the backup information from the other computer if the first mode is selected; and
controlling the analyzer if the second mode is selected.

37. A control method for a specimen analysis system, the control method being for executing, by a backup computer, obtaining of backup information about controlling of an analyzer, the control method comprising:
activating a main computer connected to the analyzer and configured to control the analyzer; and
obtaining, from the activated main computer, the backup information corresponding to information stored in the main computer.

38. The control method for the specimen analysis system, of claim 37, the control method further comprising
giving an instruction to shut down the backup computer after the backup computer activates the main computer.

39. The control method for the specimen analysis system, of claim 37 or 38, wherein the main computer
receives setting of a schedule according to which the main computer is to be activated, and
sets the received schedule with respect to the backup computer.

40. The control method for the specimen analysis system, of any one of claims 37 to 39, wherein
an activation command to activate the backup computer is transmitted from the activated main computer to the backup computer, and
the backup computer is activated when the backup computer receives the activation command.

41. The control method for the specimen analysis system, of claim 40, wherein
the main computer receives: setting of a schedule according to which the backup computer is to be activated; and setting of a schedule according to which the main computer is to be activated.

42. A specimen analysis system configured to execute, by a backup computer, obtaining of backup information about controlling of an analyzer, the specimen analysis system comprising:
an analyzer configured to analyze a specimen;
a main computer configured to control the analyzer; and
a backup computer communicably connected to the main computer, wherein
the backup computer comprises a controller, and
the controller executes
activating the main computer, and
obtaining, from the activated main computer, the backup information corresponding to information stored in the main computer.
